# EUROPEAN PATENT APPLICATION

(11) **EP 0 924 846 A2**
(43) Date of publication of application: **23.06.1999**
(21) Application number: 98310238.5
(22) Date of filing: 14.12.1998
(51) Int. Cl.: H02N 11/00

(54) **Process for producing an actuator element and microdevice**

(30) Priority: 15.12.1997 JP 34480797; 09.01.1998 JP 276498; 09.01.1998 JP 276598
(71) Applicant: AGENCY OF INDUSTRIAL SCIENCE AND TECHNOLOGY, MINISTRY OF INTERNATIONAL TRADE AND INDUSTRY, Chiyoda-ku, Tokyo 100-8921 (JP); Kaneka Medix Corporation, Osaka-shi, Osaka 530-8288 (JP)
(72) Inventor: Oguro, Keisuke, C/o Osaka Nat. Research Institute, Ikeda-shi, Osaka 563-8577 (JP); Asaka, Kinji, C/o Osaka Nat. Research Institute, Ikeda-shi, Osaka 563-8577 (JP); Fujiwara, Naoko, c/o Osaka Nat. Research Institute, Ikeda-shi, Osaka 563-8577 (JP); Sewa, Shingo, Ashigarakami-gun, Kanagawa 259-0151 (JP); Onishi, Kazuo, Amagasaki-shi, Hyogo 661-0032 (JP)
(74) Representative: Calamita, Roberto

(57) **Abstract**

A process for producing an actuator element, the actuator element comprising an ion exchange resin molding and, formed on both surfaces of the ion exchange resin molding, electrodes, the ion exchange resin molding adapted to be curved or deformed upon application of a potential difference to the ion exchange resin molding while the ion exchange resin molding is in hydrous state, which process comprises the steps of:
(1) causing the ion exchange resin molding to adsorb a metal complex in an aqueous solution, and
(2) reducing the adsorbed metal complex with a reducing agent so that a metal is precipitated on the surface of the ion exchange resin molding to thereby form a metal electrode.

## Description

The present invention relates to a process for producing an actuator element. More particularly, the present invention relates to a process for producing an actuator element capable of curving or deforming an ion exchange resin molding. Further, the present invention relates to a process for producing a lead wire which is electrically connected to an electrically operating device such as any of various sensors, a motor or an electrostatic or a polymeric actuator so that the lead wire is most suitably used in the transmission or receiving of signals and the supply of power. This electrically working device arranged at an end of an instrument inserted in an organ, such as a catheter used, for example, to inject a medicinal liquid into a blood vessel or other hollow organs or to insert a tool for observation and treatment, a guide wire inserted through the inside of the catheter and used to guide the catheter to the target site or a medical tube for use in an endoscope, etc.; or arranged at an end of a guide for use in the inspection and repair of the piping and equipment set in industrial facilities, buildings, etc. Also, the present invention relates to a working jig therefor.

Still further, the present invention relates to a microdevice for use in the inspection and repair of the piping and branched pipeline disposed in industrial facilities and equipment and buildings, and a microdevice for use in the examination and treatment in the medical field, for example, a microdevice such as forceps, scissors, a clamp, a snare, a laser knife, a spatula or a clip for use in microsurgery such as ophthalmic surgery or peritoneoscopic surgery. In particular, the present invention relates to a microdevice so constructed that it can be oscillated in arbitrary directions. Moreover, the present invention relates to a micromachine provided with the above microdevice.

Recently, there is an enhanced demand for a miniaturized, lightweight and highly flexible actuator in the fields of medical equipment, industrial robots, micromachines and the like.

When the actuator is thus miniaturized, the friction and viscous force are dominant over the inertial force. Therefore, it has been difficult to employ the means for converting energy to motion with the use of inertial force, such as a motor or an engine, as the power source of a microactuator. Accordingly, the operating principles based on electrostatic attraction, piezoelectricity, ultrasonic wave, shape memory alloy and polymer expansion/contraction have been proposed for the microactuator.

The actuator of the electrostatic attraction type operates by attracting, for example, a plate or rod becoming an electrode toward a counter electrode, and, for example, one which bends an electrode by applying a voltage of about 100 V between the electrode and the counter electrode disposed with a spacing of about tens of microns is known. The piezoelectric actuator operates by applying a voltage of some volts to a piezoelectric element of a ceramic such as barium titanate so that the element is expanded and contracted, and one capable of controlling a nm-unit displacement is known. The ultrasonic actuator operates by combining frictional force with the ultrasonic vibration generated by the piezoelectric element or the like, or by effecting a runoff. The actuator of the shape memory alloy type operates by temperature change with the use of the marked change of the configuration of the shape memory alloy depending on temperature. The actuator of the polymer expansion/contraction type operates with the use of the expansion/contraction of the polymer depending on the temperature or change of pH and change of the concentration of environmental chemical substance.

However, these microactuators have drawbacks in that there is restriction in their respective operation environments, the response is unsatisfactory, the structure is complicated and the flexibility is poor. For example, for the operation of the actuator of the polymer expansion/contraction type, the solution in contact with the polymer must be replaced by the solution containing other salt. Therefore, it has been difficult to employ this actuator in the use requiring a small size and a rapid response. In contrast, an actuator element comprising an ion exchange membrane and electrodes coupled to surfaces of the ion exchange membrane and adapted to apply a potential difference to the ion exchange membrane in the hydrous state so that the ion exchange membrane is curved or deformed has been proposed as one which can be easily miniaturized, realizes rapid response and operates with small electric power (see Japanese Patent Laid-open Publication No. 4(1992)-275078).

This actuator element is characterized by comprising an ion exchange resin membrane and metal electrodes coupled to surfaces thereof in mutually insulating relationship and by being adapted to apply a potential difference between the metal electrodes while the ion exchange resin membrane is in the hydrous state so that the ion exchange resin membrane (as ion exchange resin molding) is curved or deformed.

In this actuator element, the electrodes are formed on the surfaces of the ion exchange resin molding by chemical plating, electroplating, vacuum deposition, sputtering, coating, press bonding, fusion bonding or other methods. For example, in the chemical plating, the surfaces of the ion exchange membrane are subjected to etching, bearing of a plating catalyst and immersion in a plating bath, so that the surfaces of the ion exchange membrane are plated to thereby have electrodes formed thereon.

However, the actuator element having the electrodes formed by the above methods has a drawback in that the displacement level is not satisfactory. Therefore, it has been desired to develop an actuator element capable of realizing a greater displacement level and ensuring rapid response.

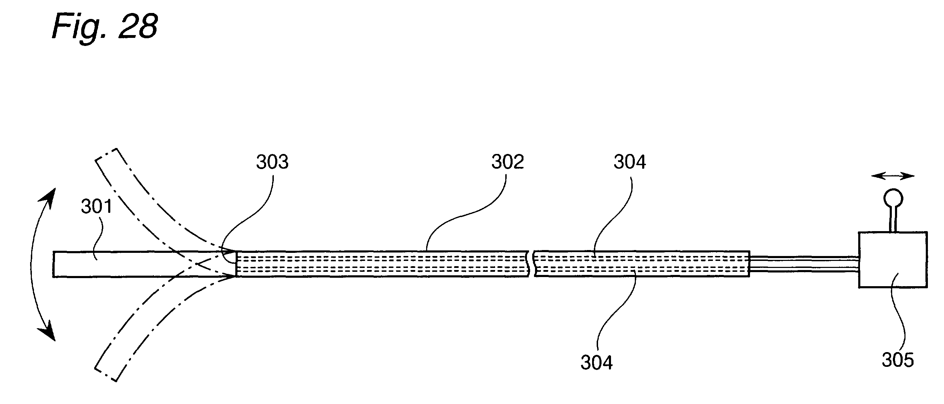

On the other hand, as shown in Fig. 28, it is common practice to arrange microdevice 301, such as any of various sensors, a motor or an actuator capable of
carrying out mechanical operation and mechanism, e.g., an electrostatic actuator, at front end portion 303 of guide 302, such as a medical tube or a medical wire, of, for example, a catheter or an endoscope for the purpose of performing treatment and observation in a hollow organ, such as an intricate blood vessel. In order to supply electric power to the above microdevice 301 or to carry out the transmission and receiving of signals, electric wire (lead wire) 304 comprising a copper wire of small diameter furnished with an insulating coating of a resin is arranged inside the tube or wire 302 or along the inner or outer wall thereof. The electric wire 304 is connected to control operating unit 305 such as an analyzer.

Also, the reliability enhancement and maintenance easiness are now major tasks in accordance with the upgrading and complication of plants, such as power generating facilities, and mechanical systems, such as aircraft engines. A microdevice such as any of various sensors or an actuator is arranged at an end of a tube or wire as a guide in order to carry out, for example, inspection or repair, without disassembly, in an extremely limited space, such as the inside of a plant piping system or aircraft engine. In this application as well, the same construction as in the medical application is employed.

The above guide may be inserted in, for example, a vital peripheral blood vessel or an intricate branched pipe of an equipment piping. Therefore, it is now required to reduce the diameter and size of the guide.

However, the above arrangement of a lead wire comprising a copper wire furnished with an insulating coating of a resin inside a tube as a guide has drawbacks in that, for meeting and sustaining the objective to inject a medicinal liquid into a blood vessel or to use in observation and treatment, the size of the guide is likely to become too large because of the need to secure, for example, a medicinal liquid path, or the medicinal liquid path inside the tube becomes too narrow, thereby lowering the effect of injecting the medicinal liquid. Moreover, fitting the microdevice of minute size with the lead wire involves difficult working, and arranging the lead wire of small diameter in the tube requires extremely precision and accurate working, thereby causing a productivity lowering.

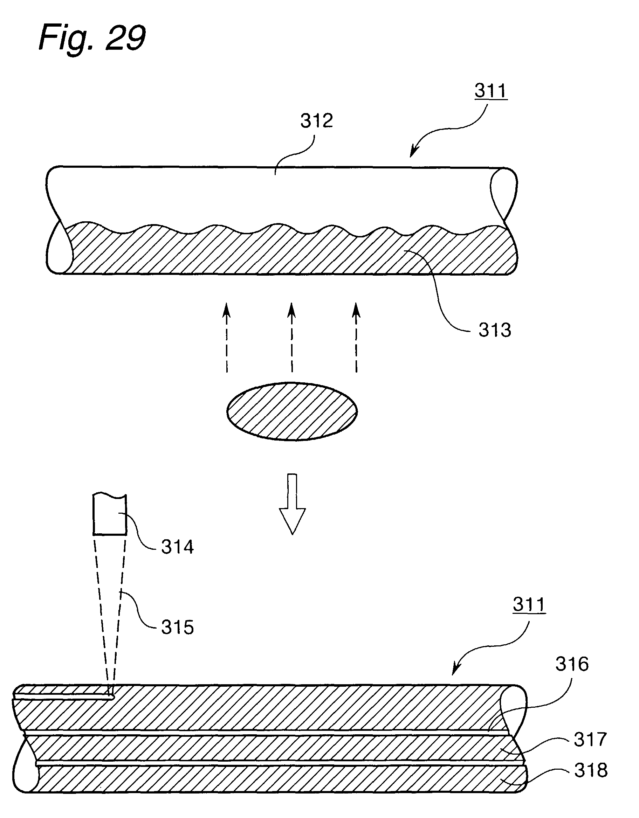

Therefore, a method has been proposed which, referring to Fig. 29, comprises coating the surface (outer wall) 312 of substrate 311 of a tube or wire as an intraorgan insertion tool with a conductive material such as a metal by the ion assist vapor deposition process to thereby form deposition coating 313 and,
thereafter, repeatedly scanning the surface of the substrate having its outer wall coated with the conductor, along the longitudinal direction of the substrate 311, with laser beam machine 314 using, for example, excimer laser so that the surface of the substrate 311 is irradiated with laser beam 315 to thereby remove parts of the coating and, hence, form insulating channels 316 with the result that a plurality of conductive paths 317, 318 in mutually insulated relationship are formed on the surface of the substrate 311 (see Japanese Patent Laid-open Publication No. 8(1996) -131545). In the method of Japanese Patent Laid-open Publication No. 8(1996)-131545, the conductive paths 317, 318 on the upper surfaces thereof are plated with a metal by the electroplating or electroless plating so that the electric resistance of the conductive paths 317, 318 is lowered.

However, this method, in which the substrate surface is coated with the conductor by the vapor deposition of a metal, requires a large apparatus for carrying out the vapor deposition of a metal, such as a vacuum deposition apparatus, and the vapor deposition operation is complicated. Therefore, the method is not suitable for continuous production. Further, the control of the bonding strength between the vapor deposition layer of a metal and the substrate surface is difficult, and the flexibility of the vapor deposition layer or plated layer is so poor that the followup thereof to substrate elongation is not satisfactory. Therefore, there has been a problem such that a layer separation from the substrate surface is caused by the insertion or push in an intricate or branched minute target site or the withdrawal therefrom or by the rotational torque with the result that the conductivity is lowered.

Moreover, in the method disclosed in Japanese Patent Laid-open Publication No. 8(1996)-131545, the insulating channels 316 are formed by repeatedly scanning the substrate 311, along the longitudinal direction thereof, with the laser beam machine 314 so that the surface of the substrate 311 is irradiated with the laser beam 315 to thereby remove parts of the coating. Therefore, unless laser irradiation is performed while accurately positioning and fixing the substrate, there would be the danger of short circuiting of conductive paths. However, in the Japanese Patent Laid-open Publication No. 8(1996)-131545, there is no disclosure teaching or suggesting the method of positioning and fixing the substrate.

On the other hand, in the contemporary medical field, there is a demand for high medical technology for minimizing the pain inflicted on patients at the time of examination or treatment to thereby relieve the physical and psychic burden on patients. The development of medical equipment for meeting the above demand is indispensable.

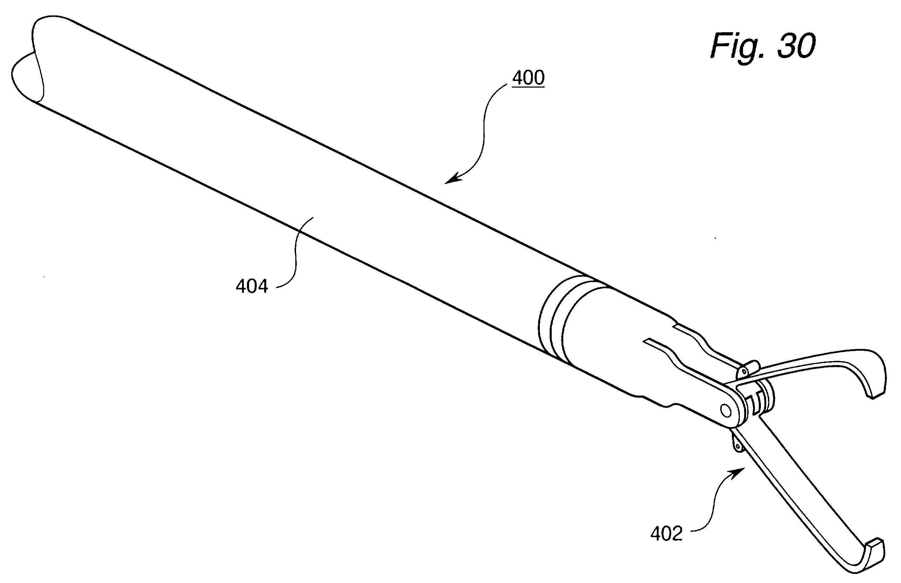

In the technology of microsurgery such as ophthalmosurgery, peritoneoscopic surgery or microangiosuturing surgery, as shown in Fig. 30, microdevice 400 comprising slender guide member body 404 constituting an intraorgan insertion part and, connected to an end thereof, therapeutic microtool 402 such as forceps, scissors, a clamp, a snare, a laser knife, a spatula or a clip has been provided as the above medical equipment. This microdevice is independently used as medical equipment.

The guide member body 404 of the microdevice 400 is composed of a tube of thermoplastic resin with some flexibility and, according to necessity, has its circumferential surface wound by a metal wire (generally, stainless steel wire) in order to increase the rigidity thereof. The microtool 402 such as a therapeutic knife connected to an end of the guide member body 404 is guided to lesion, and the lesion is extirpated or resected thereby. At the insertion of the microdevice in an organ, the end portion thereof is inserted in an intricate or branched minute part and guided to the target site while manually manipulating the end position by transmitting a rotational torque or pushing or pulling the endoscope body by means of the operating part on the handy side.

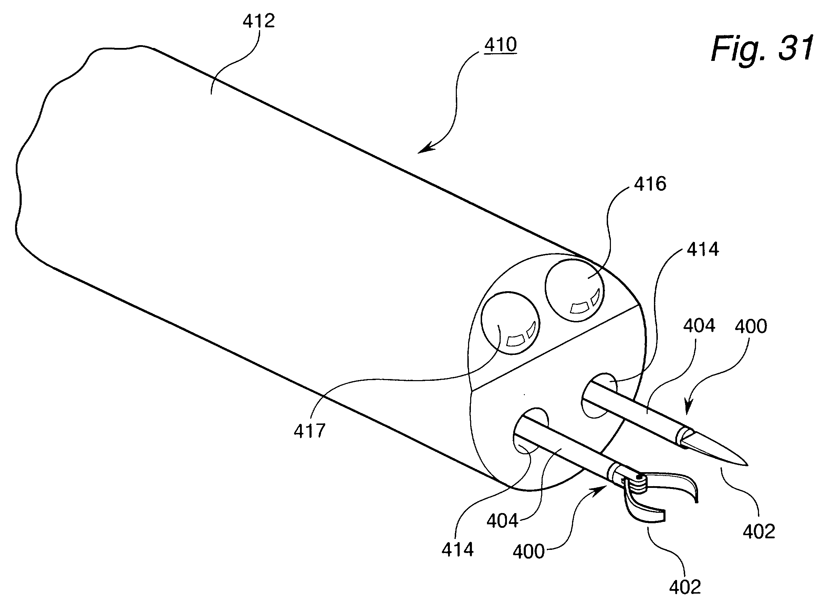

Also, referring to Fig. 31, the above microdevice 400 may be fitted in internal space for microdevice 414 of a mounting hole formed inside tubular endoscope body 412 and may be used as micromachine 410.

In this micromachine, the endoscope body 412 is also composed of a tube of thermoplastic resin with some flexibility and, according to necessity, has its circumferential surface wound by a metal wire (generally, stainless steel wire) in order to increase the rigidity thereof. For example, at the insertion of the endoscope body 412 in an organ, the end portion thereof is inserted in an intricate or branched minute part and guided to the target site while manually manipulating the end position by transmitting a rotational torque or pushing or pulling the endoscope body by means of the operating part on the handy side. Upon the guidance of the endoscope body 412 to the target site, the therapeutic microtool 402 of the microdevice 400 and the end of the guide member body 404 thereof are protruded from the internal space for microdevice 414. The therapeutic microtool 402 such as a therapeutic knife connected to the end of the guide member body 404 is guided to lesion, and the lesion is extirpated or resected thereby. In Fig. 31, numeral 416 denotes an irradiation lens and numeral 417 a photographic lens.

On the other hand, the reliability enhancement and maintenance easiness are now major tasks in accordance with the upgrading and complication of plants, such as power generating facilities, and mechanical systems, such as aircraft engines. Thus, when, for example, inspection or repair is performed, without disassembly, in an extremely limited space, such as the inside of a plant piping system or aircraft engine, use is also made of a microdevice with similar structure, comprising a slender guide member body and, connected to an end thereof, a microtool such as any of various sensors or a repair tool. Further, use is made of a micromachine furnished with the microdevice.

However, with respect to the independent use of the microdevice of the above structure, for example, in ophthalmic surgery, the outer diameter of the guide member body constituting the intraorgan insertion part of the microdevice is about 0.9 mm, and the guide member body has a straight form. Therefore, the freedom thereof is low, and it is difficult to guide the microtool, such as a therapeutic knife, connected to an end of the guide member body to lesion or the like to thereby resect or extirpate the same. The operator must have high degrees of expertise and experience, and there is limitation in the manual skill with the result that physical and psychic burden may be inflicted on the patients.

On the other hand, with respect to the use of the microdevice fitted in the mounting hole provided inside the endoscope body as a micromachine, similar problem is encountered in the operation of leading the endoscope body to the target site in an organ and, thereafter, guiding the microtool such as a therapeutic knife connected to an end of the guide member body to lesion to thereby resect or extirpate the same.

These are the same in, for example, a piping system or engine interior of plants, such as power generating facilities, and mechanical systems, such as aircraft engines, so that the consumption of labor and time has been substantial in the repairing thereof.

The present invention is intended to solve the above problems of the prior art. Accordingly, it is an object of the present invention to provide a process for producing an actuator element which exhibits a large displacement extent and a large displacement force, has a simple structure, can be easily miniaturized, exhibits a high response speed and is flexible.

It is another object of the present invention to provide a process for producing a lead wire, in which conductive lines of a conductive coating can continuously be applied to a surface of a lead wire substrate such as a tube or wire efficiently by simple procedure without the need to use a large apparatus. It is still another object of the present invention to provide a process for producing a highly conductive lead wire which comprises a substrate and conductive lines bonded to a surface thereof with a high strength, the conductive lines being excellent in flexibility, being excellent in the follow-up to substrate elongation and not peeled from the substrate surface by the insertion or push in intricate or branched minute target sites or the pull therefrom or by the rotational torque.

It is a further object of the present invention to provide a process for producing a lead wire, in which, in the formation of insulating zones conducted by irradiating a surface of a lead wire substrate with, for example, laser beam to thereby remove part of a coating, the irradiation with laser, etc. can be performed with the substrate accurately positioned and fixed to thereby enable avoiding, for example, mutual short circuiting of conductive lines. It is still a further object of the present invention to provide a working jig therefor. It is still a further object of the present invention to provide a microdevice which realizes freely guiding a microtool connected to an end of a guide member body to, for example, lesion in an organ or breakdown part of a piping of mechanical system to thereby enable facilitating and perfectly carrying out an appropriate surgery or repair and to provide a micromachine furnished with the microdevice.

The present invention has been made with a view toward solving the above problems of the prior art and attaining the above objects. Therefore, in one aspect of the present invention, there is provided a process for producing an actuator element, the above actuator element comprising an ion exchange resin molding and metal electrodes which are formed in mutually insulated relationship on surface of the ion exchange resin molding, the above ion exchange resin molding adapted to be curved or deformed upon application of a potential difference between the metal electrodes while the ion exchange resin molding is in hydrous state, which process comprises the steps of:
(1) causing the ion exchange resin molding to adsorb a metal complex in an aqueous solution, and
(2) reducing the adsorbed metal complex with a reducing agent so that a metal is precipitated on surface of the ion exchange resin molding to thereby form a metal electrode.

The formation of a metal electrode by the above process enables obtaining an actuator element which has a simple structure, can be easily miniaturized, exhibits a high response speed and exhibits a large displacement extent.

It is preferred that a gold or platinum complex be used as the metal complex. The use of a gold or platinum complex enables obtaining an actuator element exhibiting a large displacement extent.

In the present invention, upon formation of the metal electrode, the ion exchange resin molding preferably has its counter ion replaced by at least one cation selected from the group consisting of Li⁺, Na⁺ and Cu²⁺. The displacement extent of the obtained actuator element can further be increased by the cation replacement.

The process for producing a lead wire according to the present invention comprises the steps of:
coating surface of a lead wire substrate of an insulating material with a conductive solution to thereby form a conductive thin-film layer on the surface, and
   irradiating the lead wire substrate, in one direction therealong, with light so that the conductive thin-film layer is removed by the irradiation working to thereby form insulating zones and thus form a plurality of mutually electrically insulated conductive lines along the lead wire substrate.

In this process, it is preferred that the coating with the conductive solution be performed by a dip coating and that the conductive solution be a conductive solution of Au or Ag.

This process, because it is only required to coat the surface of the lead wire substrate with the conductive solution, enables continuously providing conductive lines efficiently by simple procedure without the need to use a large apparatus. Furthermore, a highly conductive lead wire can be provided thereby, in which the bonding strength between conductive lines and substrate surface is excellent, the conductive lines being excellent in flexibility, being excellent in the follow-up to substrate elongation and not peeled from the substrate surface by the insertion or push in intricate or branched minute target sites or the pull therefrom or by the rotational torque.

It is preferred that the lead wire substrate be in tubular form. In this instance, the lead wire per se can be used as a guide, such as a medical tube or a medical wire, for a catheter, an endoscope, etc., so that a medicinal liquid path can be ensured and a size miniaturization can be attained. Further, in the fitting of the above lead wire in a fine microdevice, the lead wire fitting operation is easy to thereby ensure high productivity.

It is preferred that the irradiation working be performed by laser from the viewpoint that noncontacting working can be effected and, hence, an outer wall having a curved surface such as that in tubular form can be easily worked.

Moreover, the process for producing a lead wire according to the present invention is characterized in that, in the irradiation working, the lead wire substrate is fitted and fixed in a fixing channel provided in an irradiation working jig, and surface of the lead wire substrate, which is exposed from the irradiation working jig, is subjected to the irradiation working.

This enables providing a lead wire in which mutual short circuiting of the conductive lines can be avoided because, in the formation of insulating zones by partial removal of the coating by irradiating the surface of the lead wire substrate with, for example, laser beam, the formation of the insulating zones can be performed with the lead wire substrate accurately positioned and fixed.

It is preferred that the irradiation working jig comprise a plate base frame having its upper surface provided with a fixing channel, this fixing channel adapted to fix a lead wire substrate so that a surface of the lead wire substrate is subjected to irradiation from upside. This construction enables securely positioning and fixing the lead wire substrate in the fixing channel.

In the present invention, preferably, the irradiation working jig comprises a pair of base frames arranged upside and downside, these base frames having matching faces provided with fixing channels, the above base frames provided with irradiation slits extending from the fixing channels to upper and lower surfaces of the base frames, and the lead wire substrate is fixed between the fixing channels by closing the base frames together and is at its surface subjected to irradiation working through the irradiation slits. This enables accurately forming two insulating zones at the upper and lower surfaces of the lead wire substrate, respectively.

Further, preferably, the irradiation working jig comprises four base frames arranged upside right, upside left, downside right and downside left, these base frames having matching faces provided with fixing channels, the above base frames provided with irradiation slits extending from upper, right, lower and left surfaces of the base frames to the fixing channels, and the lead wire substrate is fixed between the fixing channels by closing the base frames together and the lead wire substrate is at its surface subjected to irradiation working through the irradiation slits. This enables accurately forming a total of four insulating zones at the upper, right, lower and left surfaces of the lead wire substrate, respectively.

The working jig of the present invention is a jig for irradiation working for use in producing a lead wire, comprising a plate base frame having its upper surface provided with a fixing channel, this fixing channel adapted to fix a lead wire substrate so that a surface of the lead wire substrate is subjected to irradiation from upside.

This enables providing a lead wire in which mutual short circuiting of the conductive lines can be avoided because, in the formation of insulating zones by partial removal of the coating by irradiating the surface of the lead wire substrate with, for example, laser beam, the formation of the insulating zones can be performed with the lead wire substrate accurately positioned and fixed. It is preferred that the fixing channel be defined by a fixed plate fastened to an upper surface of the base frame and a channel width regulating plate arranged, movably toward the fixed plate, on the upper surface of the base frame. The reason for the preference is that the lead wire substrate can be securely fixed.

Further, the working jig of the present invention may comprise a pair of base frames arranged upside and downside,
the above base frames having matching faces provided with fixing channels, the above fixing channels adapted to fix a lead wire substrate therebetween by closing the base frames together,
the above base frames provided with irradiation slits extending from upper and lower surfaces of the base frames to the fixing channels,
the above irradiation slits adapted to perform irradiation working of a surface of the lead wire substrate therethrough. This enables accurately forming two insulating zones at the upper and lower surfaces of the lead wire substrate, respectively.

Still further, the working jig of the present invention may comprise four base frames arranged upside right, upside left, downside right and downside left,
the above base frames having matching faces provided with fixing channels, the above fixing channels adapted to fix a lead wire substrate therebetween by closing the base frames together,
the above base frames provided with irradiation slits extending from upper, right, lower and left surfaces of the base frames to the fixing channels, the above irradiation slits adapted to perform irradiation working of a surface of the lead wire substrate therethrough. This enables accurately forming a total of four insulating zones at the upper, right, lower and left surfaces of the lead wire substrate, respectively.

The microdevice of the present invention comprises:
a slender guide member body;
   a therapeutic tool connected to a front end of the guide member body;
   a polymeric actuator arranged in the vicinity of part of the connection; and
   an actuator operation control unit electrically connected to the actuator through a lead wire extending along a longitudinal direction of the guide member body,
      wherein the polymeric actuator comprises an ion exchange resin molding and at least one pair of electrodes, provided in positions interposing the ion exchange resin molding, and
      wherein an arrangement is made such that the ion exchange resin molding is deformed by selective application of a voltage through the lead wire to the pair of electrodes by means of the operation control unit so that the therapeutic tool connected to the front end of the guide member body can be oscillated in any arbitrary direction.

Moreover, the micromachine of the present invention comprises the above microdevice fitted in an internal space of a tubular slender guide member body, wherein an arrangement is such that the microdevice can be protruded through an opening formed at a front end of the slender guide member body.

When the microdevice is inserted in an organ or a piping system of mechanical system and guided to the target site, by virtue of the above arrangement, the therapeutic tool connected to the front end of the guide member body can be oscillated in any arbitrary direction, so that it does not become a hindrance and is free from inadvertently injuring the organ interior or piping. Furthermore, when the microdevice must be operated, it can be guided to lesion in an organ and failure site in a piping system of mechanical system, so that the operation and repair can be carried out easily and securely.

In the accompanying drawings:
Fig. 1 is a schematic sectional view of one form of actuator element obtained by the process of the present invention, which is in the state of having no voltage applied thereto;
Fig. 2 is a schematic sectional view of one form of actuator element obtained by the process of the present invention, which is in the state of having a voltage applied thereto;
Fig. 3 is a schematic diagram showing an example of application of one form of actuator element obtained by the process of the present invention;
Fig. 4 is a schematic enlarged view of an essential part of Fig. 3;
Fig. 5 is a schematic diagram showing another form of actuator element obtained by the process of the present invention;
Fig. 6 is a schematic diagram explaining one mode of step of coating with a conductive solution, conducted in the process for producing a lead wire according to the present invention;
Fig. 7 is a schematic diagram explaining one mode of step of forming insulating zones by irradiation working, conducted in the process for producing a lead wire according to the present invention;
Fig. 8 is a schematic diagram explaining another mode of step of coating with a conductive solution, conducted in the process for producing a lead wire according to the present invention;
Fig. 9 is a perspective view of one form of working jig for use in the process for producing a lead wire according to the present invention;
Fig. 10 is a perspective view of another form of working jig for use in the process for producing a lead wire according to the present invention;
Fig. 11 is a perspective view of still another form of working jig for use in the process for producing a lead wire according to the present invention;
Fig. 12 is a perspective view of a further form of working jig for use in the process for producing a lead wire according to the present invention;
Fig. 13 is a sectional view on the line A-A of Fig. 12;
Fig. 14 is a partially enlarged sectional view of still a further form of working jig for use in the process for producing a lead wire according to the present invention;
Fig. 15 is a perspective view of still a further form of working jig for use in the process for producing a lead wire according to the present invention;
Fig. 16 is a sectional view on the line B-B of Fig. 15;
Fig. 17 is a schematic diagram of another form of irradiation working apparatus for use in the present invention;
Fig. 18 is a partially cutaway perspective view of the first form of micromachine fitted with the microdevice of the present invention applied to the medical field, especially, microsurgery field;
Fig. 19 is a schematic diagram of one form of microdevice of the present invention;
Fig. 20 is a schematic diagram of an essential part of the microdevice of the present invention shown in Fig. 19;
Fig. 21 is a schematic diagram showing the micromachine furnished with one form of microdevice of the present invention which is in the state of resecting and extirpating lesion in an organ;
Fig. 22 is an enlarged perspective view of an essential part of another form of microdevice of the present invention;
Fig. 23 is a schematic view of still another form of microdevice of the present invention;
Fig. 24 is a schematic diagram of an essential part of the microdevice of the present invention shown in Fig. 23;
Fig. 25 is an enlarged perspective view of an essential part of a further form of microdevice of the present invention;
Fig. 26 is an enlarged sectional view on the line C-C of Fig. 25;
Fig. 27 is an enlarged sectional view on the line D-D of Fig. 25;
Fig. 28 is a schematic diagram of the conventional medical tube;
Fig. 29 is a schematic diagram explaining the process for producing the conventional lead wire;
Fig. 30 is a perspective view of the conventional microdevice; and
Fig. 31 is a perspective view of the micromachine provided with the conventional microdevice.

Embodiments of the present invention will be described in detail below with reference to the drawings.

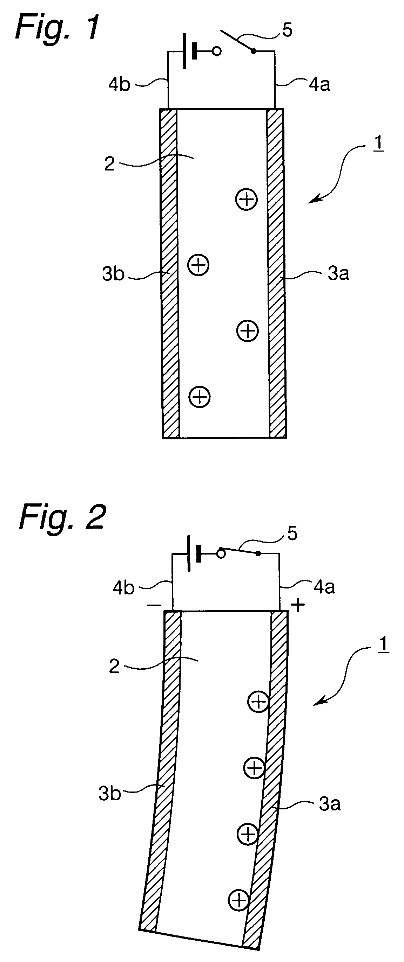

Figs. 1 and 2 are schematic sectional views of the most suitable form of actuator element obtained by the process of the present invention. In this form of actuator element, actuator element 1 comprises slender rectangular plate of ion exchange resin molding 2 and electrodes 3a, 3b arranged in mutually insulating relationship on opposite surfaces of the ion exchange resin molding 2. A potential difference is applied between the electrodes 3a, 3b while the ion exchange resin molding 2 is in a hydrous state, so that the ion exchange resin molding 2 is curved or deformed. Ends of a pair of lead wires 4a, 4b are respectively electrically connected to the electrodes 3a, 3b, and the other ends of the lead wires 4a, 4b are connected to power source 5.

The shape of the ion exchange resin molding 2 is not limited to the above rectangular plate, and may be, for example, a membrane, a column or a cylinder. The ion exchange resin for forming the above ion exchange resin molding 2 can be an anion exchange resin, a cation exchange resin or an amphoteric ion exchange resin. Of these, the cation exchange resin is preferably used because the extent of displacement of the actuator element can be large.

The above cation exchange resin can be a resin such as polyethylene, polystyrene or a fluororesin having, introduced therein, a functional group such as a sulfonate group or a carboxyl group. Especially, a cation exchange resin comprising a fluororesin having, introduced therein, a functional group such as a sulfonate group or a carboxyl group is preferred. Among the above cation exchange resins, those having an ion exchange capacity of 0.8 to 2.2 meq/g, especially, 1.4 to 1.8 meq/g are still preferred. The use of the cation exchange resin having such an ion exchange capacity enables further increasing the extent of displacement of the actuator element.

In the present invention, a metal complex is adsorbed, on the ion exchange resin molding in an aqueous solution, and the metal complex having been adsorbed on the ion exchange resin molding is reduced by a reducing agent to thereby precipitate a metal on surfaces of the ion exchange resin molding. Thus, metal electrodes are formed.

As this metal complex, any of gold, platinum, palladium, rhodium and ruthenium complexes can be used. Of these, gold and platinum complexes are preferred because the extent of displacement of the actuator element can be large.

The adsorption of the above metal complex onto the ion exchange resin molding is effected by immersing the ion exchange resin molding in an aqueous solution containing the metal complex.

The reduction of the metal complex is effected by immersing the ion exchange resin molding having the metal complex adsorbed thereonto in an aqueous solution containing a reducing agent.

Although depending on the type of employed metal complex, the reducing agent can be selected from among, for example, sodium sulfite, hydroxylamine hydrochloride, hydrazine and potassium borohydride. According to necessity, an acid or an alkali may be added in the reduction of the metal complex.

Upon the reduction of the metal complex adsorbed on the ion exchange resin molding, a metal precipitates on surfaces of the ion exchange resin molding, thereby forming metal electrodes.

In the present invention, surfaces of the employed ion exchange resin molding may be roughened prior to the formation of the metal electrodes. The roughening of membrane surface can be conducted by the use of, for example, sandblasting or sand paper treatment. The degree of the roughening of the surface may be such that the surface layer is shaved.

This roughening increases the area of contact of the surfaces of the ion exchange resin molding and the later formed electrodes to thereby enable increasing the extent of displacement of the actuator element. Further, in the present invention, the employed ion exchange resin molding may be subjected to:
(1) water treatment in which the ion exchange resin molding is heated in boiling water;
(2) hydrochloric acid treatment in which the ion exchange resin molding is held in dilute hydrochloric acid of about 25% by volume;
(3) NaOH treatment in which the ion exchange resin molding is held in an aqueous sodium hydroxide solution of about 0.1 N;
(4) alcohol treatment in which the ion exchange resin molding is immersed in an alcohol such as methanol or ethanol; or
(5) autoclave treatment in which the ion exchange resin molding is heated at 110 to 150°C in an autoclave.

With respect to the reduction of the metal complex adsorbed on the ion exchange resin molding, it is presumed that a metal precipitation occurs at the surface of the ion exchange resin molding at which the metal complex contacts the reducing agent and, accordingly, the metal complex lying in the internal part of the membrane moves toward the vicinity of the membrane surface (toward the precipitated metal) and is reduced to thereby attain the desired metal precipitation. The metal precipitation is made in not only the surface of the ion exchange resin molding but also the internal part close to the surface, so that the area of contact of the ion exchange resin molding and the metal electrode would be larger than in the conventional chemical plating process. Therefore, the actuator element obtained in the present invention exhibits a greater extent of element displacement than that of the conventional actuator element.

After the electrode formation, the ion exchange resin molding is generally washed with pure water to thereby remove unprecipitated metal complex and the reducing agent. The insulation between the electrodes can be effected by cutting the edges of the ion exchange resin molding having the metal electrodes formed thereon. Further, the insulation between the electrodes can be effected by irradiating the ion exchange resin molding having the metal electrodes formed thereon with laser beam or electron beam to thereby shave parts of the metal electrodes with the result that insulating zones are disposed between the electrodes.

Moreover, the ion exchange resin molding having the electrodes formed thereon may be subjected to the above treatments (1) to (5).

Furthermore, additional electrode layers may be disposed on the formed electrodes. The additional electrode layers can be formed by chemical plating, electroplating, vacuum deposition, sputtering, coating, press bonding, fusion bonding or other methods. These additional electrode layers may be either identical with or different from the metal layers formed on the surfaces of the ion exchange resin molding.

Still further, the ion exchange resin molding having the electrodes formed thereon may have its counter ion replaced by, for example, Li⁺, Na⁺, Cu²⁺, H⁺, Ca²⁺, Fe³⁺ or Mg²⁺. Of these ions, the replacement is preferably conducted by Li⁺, Na⁺ or Cu²⁺. This counter ion replacement can be effected by immersing the ion exchange resin molding having the electrodes formed thereon in an aqueous solution of LiOH, NaOH, etc.

At the operation of the thus obtained actuator element, it is requisite that the ion exchange membrane should be in the hydrous state. The terminology "hydrous state" used herein means that the actuator operates in not only water but also highly humid atmosphere.

With respect to the mechanism of operation of the above actuator element, it is presumed that, when a potential difference is applied between opposite surfaces of the ion exchange resin molding which are in mutually insulating relationship, positive (+) ions 4 lying in the ion exchange resin molding move to the negative electrode side as shown in Fig. 2 and, entrained by the ions, water molecules move in the membrane with the result that a water content difference occurs between the positive electrode side and the negative electrode side. Thus, it is presumed that the increase in water content causes a swelling and the decrease in water content causes a shrinkage, so that the ion exchange resin molding is curved.

The thus obtained actuator element can have a displacement of about 0.5 to 3 times the length of the actuator element within a period of seconds at the application of a direct current voltage of 0.1 to 3 V between the electrodes. This actuator element can flexibly operate in water.

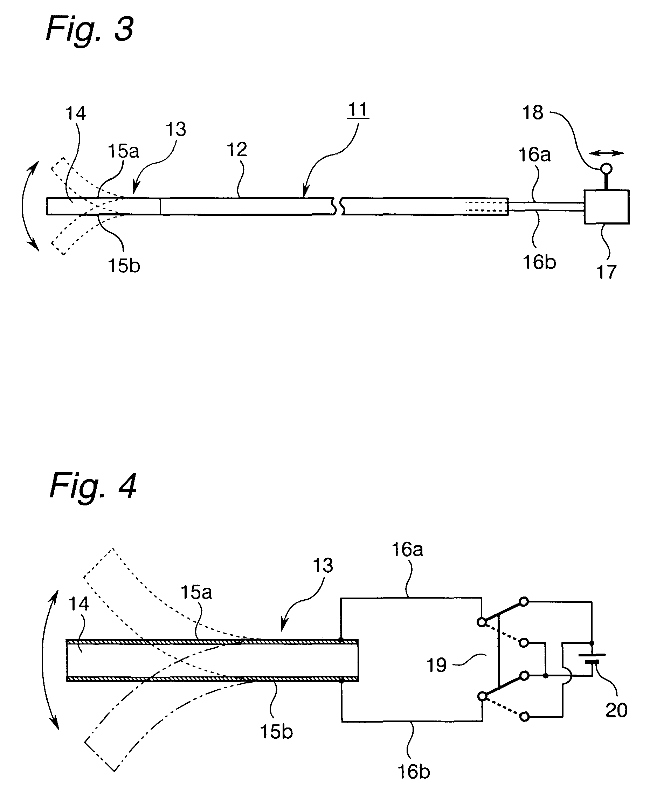

An example of application of this actuator element can be found in the guide of Fig. 3.

In this application example, guide wire 11 as a guide comprises linear member 12 composed of, for example, a slender tube of synthetic resin or stainless steel and actuator element 13 connected to an end of the linear member 12.

The above actuator element 13 comprises ion exchange resin molding 14 shaped into a relatively slender rectangular plate and, disposed on both sides thereof, a pair of electrodes 15a, 15b formed by the process of the present invention. Application of a voltage to the electrodes 15a, 15b causes the actuator element 13 to curve in two directions.

Ends of a pair of lead wires 16a, 16b are electrically connected to the electrodes 15a, 15b, respectively. The lead wires 16a, 16b are positioned inside the linear member 12 and extend along the entire length of the linear member 12, and the other ends of the lead wires 16a, 16b are connected to operation control unit 17.

This operation control unit 17 is fitted with operating lever 18 which enables switching operation. The electrode of the current from power source 20 which flows through the pair of lead wires 16a, 16b can be switched by means of double-pole double-throw switch 19 disposed inside the operation control unit 17 in accordance with the manipulation made by the operating lever 18.

That is, referring to Fig. 4, when the double-pole double-throw switch 19 is arranged in the position indicated by the full line, the lead wires 16a and 16b are connected to the positive electrode and the negative electrode, respectively. On the other hand, when the double-pole double-throw switch 19 is rearranged from the neutral position as indicated by the two-dot long and two short dashes line in accordance with the manipulation made by the operating lever 18 of the operation control unit 17, contrarily, the lead wires 16a and 16b are connected to the negative electrode and the positive electrode, respectively.

As apparent from the above, the actuator element 1 can arbitrarily and positively be deformed by manipulating the positive electrode and the negative electrode.

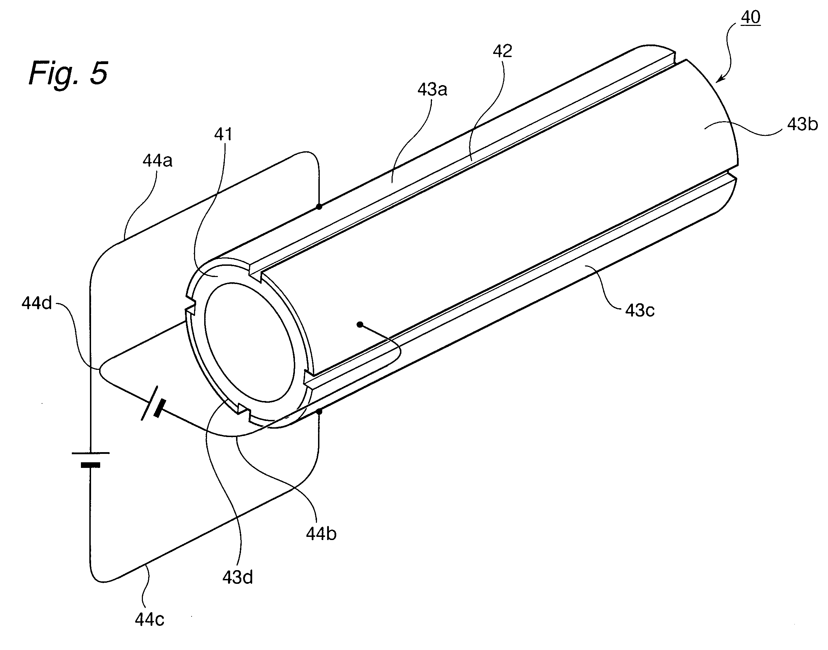

Furthermore, cylindrical actuator element 40 as shown in Fig. 5 can be produced by the process of the present invention.

In the production of this cylindrical actuator element 40, first, a metal complex is adsorbed on cylindrical ion exchange resin molding 41 by the above procedure. The metal complex is reduced by a reducing agent to thereby precipitate a metal on the surface of the ion exchange resin molding 41 so that a metal layer is formed.

Subsequently, the cylindrical ion exchange resin molding 41 having its outer surface furnished with the metal layer, at the outer surface, is irradiated with laser beam from a laser beam machine to thereby remove the metal layer at the irradiated part. Thus, channel-shaped insulating zones 42 and a plurality of mutually electrically insulated metal electrodes 43a, 43b, 43c, 43d are formed.

In the actuator element of Fig. 5, ends of lead wires 44a, 44b, 44c, 44d are electrically connected to the metal electrodes 43a, 43b, 43c, 43d, respectively. The ion exchange resin molding 41 can be curved in four directions by applying a voltage to electrodes 43a, 43c disposed opposite to each other with the ion exchange resin molding 41 interposed therebetween and to electrodes 43b, 43d disposed opposite to each other with the ion exchange resin molding 41 interposed therebetween. Further, the curving directions can be combined with each other to thereby enable a rotation. These metal electrodes may be arranged on an inner circular face of the ion exchange resin molding.

### Example 1

A 0.2 mm-thick fluororesin ion exchange resin molding (Nafion 117 produced by E.I. DuPont de Nemours & Co., Inc., having an ion exchange capacity of 0.9 meq/g) was sandblasted with the use of alumina particles (no. 800), thereby roughening the surface of the ion exchange resin molding. After the surface roughening, sandblast particles and other dirt were removed from the ion exchange resin molding by ultrasonic cleaning. Thereafter, the ion exchange resin molding was boiled in an aqueous 25% by volume hydrochloric acid solution for 30 min and boiled in pure water for 30 min to thereby remove hydrochloric acid therefrom.

After the above boiling, the ion exchange resin molding was immersed in an aqueous solution of platinum/amine complex for 6 hr to thereby cause the ion exchange resin molding at its internal part to adsorb the platinum/amine complex. Thereafter, the ion exchange resin molding was immersed in an aqueous solution of sodium borohydride at 40 to 65°C for 5 hr to thereby reduce the platinum/amine complex with the result that platinum was precipitated on the surface of the ion exchange resin molding.

The ion exchange resin molding having platinum precipitated on its surface was further immersed in an aqueous solution of platinum/amine complex, and an aqueous solution of hydroxylamine hydrochloride and an aqueous solution of hydrazine were added thereto and heated at 40 to 60°C for 5 hr to thereby reduce the platinum/amine complex. Thus, platinum growing plating was performed on the surface of the ion exchange resin molding, thereby accomplishing an electrode formation. After the electrode formation, the resultant ion exchange resin molding was boiled in an aqueous 25% by volume hydrochloric acid solution for 30 min and boiled in pure water for 30 min to thereby remove hydrochloric acid therefrom.

The above ion exchange resin molding was immersed in a 1M aqueous LiOH solution for 6 hr to thereby replace the counter ion by Li⁺ and cut into strips of 1 mm width. Thus, an actuator element was obtained. The extent of bending displacement of a front end portion exhibited when a voltage of 2 V was applied to the obtained actuator element was measured by the use of a laser displacement meter. The extent of bending displacement was evaluated in terms of the displacement extent per 10 mm of the element length.

The results are given in Table 1.

### Example 2

An actuator element was produced in the same manner as in Example 1, except that a 1M aqueous NaOH solution was used in place of the 1M aqueous LiOH solution to thereby replace the counter ion by Na⁺. The extent of bending displacement thereof was evaluated in the same manner as in Example 1. The results are given in Table 1.

### Example 3

An actuator element was produced in the same manner as in Example 1, except that an ion exchange resin molding exhibiting an ion exchange capacity of 1.4 meq/g was used, and that a 1M aqueous NaOH solution was used in place of the 1M aqueous LiOH solution to thereby replace the counter ion by Na⁺. The extent of bending displacement thereof was evaluated in the same manner as in Example 1. The results are given in Table 1.

### Example 4

An actuator element was produced in the same manner as in Example 1, except that a 1M aqueous CuSO₄ solution was used in place of the 1M aqueous LiOH solution to thereby replace the counter ion by Cu²⁺. The extent of bending displacement thereof was evaluated in the same manner as in Example 1.

The results are given in Table 1.

### Example 5

The roughened ion exchange resin molding which was obtained in the same manner as in Example 1 was boiled in an aqueous 25% by volume hydrochloric acid solution for 30 min and boiled in pure water for 30 min to thereby remove hydrochloric acid therefrom.

The resultant ion exchange resin molding was immersed in an aqueous solution of gold complex (dichlorophenanthrolinegold chloride) to thereby cause the ion exchange resin molding at its internal part to adsorb the gold complex. The ion exchange resin molding having adsorbed the gold complex was immersed in an aqueous solution of sodium sulfite and heated at 40 to 80°C for 5 hr to thereby reduce the gold complex with the result that gold was precipitated on the surface of the ion exchange resin molding to thereby attain an electrode formation.

The ion exchange resin molding, on which the electrode was formed, was immersed in 1N sulfuric acid for 30 min and further immersed in pure water to thereby remove sulfuric acid therefrom.

The above ion exchange resin molding was immersed in a 1M aqueous LiOH solution for 6 hr to thereby replace the counter ion by Li⁺ and cut into strips of 1 mm width. Electrode connection was effected, thereby obtaining an actuator element.

The extent of bending displacement of the obtained actuator element was evaluated in the same manner as in Example 1.

The results are given in Table 1.

### Comparative Example 1

The surface of the same ion exchange resin molding as employed in Example 1 was subjected to chemical plating so that a platinum electrode was formed on the surface of the ion exhange resin molding.

The ion exchange resin molding, on which the electrode was formed, was immersed in a 1M aqueous LiOH solution for 6 hr to thereby replace the counter ion by Li⁺ and cut into strips of 1 mm width, in the same manner as in Example 1. Electrode connection was effected, thereby obtaining an actuator element.

The extent of bending displacement of the obtained actuator element was evaluated in the same manner as in Example 1.

The results are given in Table 1.

### Comparative Example 2

The surface of the same ion exchange resin molding as employed in Example 1 was subjected to a vacuum deposition of platinum so that a platinum electrode was formed on the surface of the ion exchange resin molding.

The ion exchange resin molding, on which the electrode was formed, was immersed in a 1M aqueous LiOH solution for 6 hr to thereby replace the counter ion by Li⁺ and cut into strips of 1 mm width, in the same manner as in Example 1. Electrode connection was effected, thereby obtaining an actuator element.

The extent of bending displacement of the obtained actuator element was evaluated in the same manner as in Example 1.

The results are given in Table 1.

### Comparative Example 3

The surface of the same ion exchange resin molding as employed in Example 1 was subjected to sputtering so that the surface was furnished with a platinum electrode.

The ion exchange resin molding, on which the electode was formed, was immersed in a 1M aqueous LiOH solution for 6 hr to thereby replace the counter ion by Li⁺ and cut into strips of 1 mm width, in the same manner as in Example 1. Electrode connection was effected, thereby obtaining an actuator element.

The extent of bending displacement of the obtained actuator element was evaluated in the same manner as in Example 1.

The results are given in Table 1.

### Comparative Example 4

The surface of the same ion exchange resin molding as employed in Example 1 was coated with a conductive ink containing fine particles of platinum so that a platinum electrode was formed on the surface of the ion exchange resin molding.

The ion exchange resin molding, on which the electrode was formed, was immersed in a 1M aqueous LiOH solution for 6 hr to thereby replace the counter ion by Li⁺ and cut into strips of 1 mm width, in the same manner as in Example 1. Electrode connection was effected, thereby obtaining an actuator element.

The extent of bending displacement of the obtained actuator element was evaluated in the same manner as in Example 1.

The results are given in Table 1.

### Example 6

An actuator element was produced in the same manner as in Example 1, except that a 1M aqueous HCl solution was used in place of the 1M aqueous LiOH solution to thereby replace the counter ion by H⁺, and the extent of bending displacement thereof was evaluated in the same manner as in Example 1.

The results are given in Table 1.

### Example 7

An actuator element was produced in the same manner as in Example 1, except that a 1M aqueous CaCl₂ solution was used in place of the 1M aqueous LiOH solution to thereby replace the counter ion by Ca²⁺, and the extent of bending displacement thereof was evaluated in the same manner as in Example 1.

The results are given in Table 1.

### Example 8

An actuator element was produced in the same manner as in Example 1, except that a 1M aqueous FeCl₃ solution was used in place of the 1M aqueous LiOH solution to thereby replace the counter ion by Fe³⁺, and the extent of bending displacement thereof was evaluated in the same manner as in Example 1.

The results are given in Table 1.

### Example 9

An actuator element was produced in the same manner as in Example 1, except that a 1M aqueous MgCl₂ solution was used in place of the 1M aqueous LiOH solution to thereby replace the counter ion by Mg²⁺, and the extent of bending displacement thereof was evaluated in the same manner as in Example 1.

The results are given in Table 1.

### Example 10

An actuator element was produced in the same manner as in Example 1, except that the ion exchange resin molding was immersed in an aqueous solution of rhodium complex in place of the immersion in the aqueous solution of platinum/amine complex to thereby form a rhodium electrode. The extent of bending displacement thereof was evaluated in the same manner as in Example 1.

The results are given in Table 1.

**Table 1**

| | Electrode metal | Counter ion | Method of forming electrode | Extent of displacement (mm) |
|---|---|---|---|---|
| Example 1 | Pt | Li⁺ | | 3.2 |
| Example 2 | Pt | Na⁺ | | 2.8 |
| Example 3 | Pt | Na⁺ | | 4.1 |
| Example 4 | Pt | Cu²⁺ | | 2.5 |
| Example 5 | Au | Li⁺ | | 2.9 |
| Comp.Ex.1 | Pt | Li⁺ | Chemical plating | 0.2 |
| Comp.Ex.2 | Pt | li⁺ | Vacuum deposition | 0.2 |
| Comp.Ex.3 | Pt | Li⁺ | Sputtering | 0.1 |
| Comp.Ex.4 | Pt | Li⁺ | Conductive ink | 0 |
| Example 6 | Pt | H⁺ | | 1.1 |
| Example 7 | Pt | Ca²⁺ | | 0.4 |
| Example 8 | Pt | Fe³⁺ | | 0.7 |
| Example 9 | Pt | Mg²⁺ | | 0.6 |
| Example10 | Pt | Li⁺ | | 0.4 |

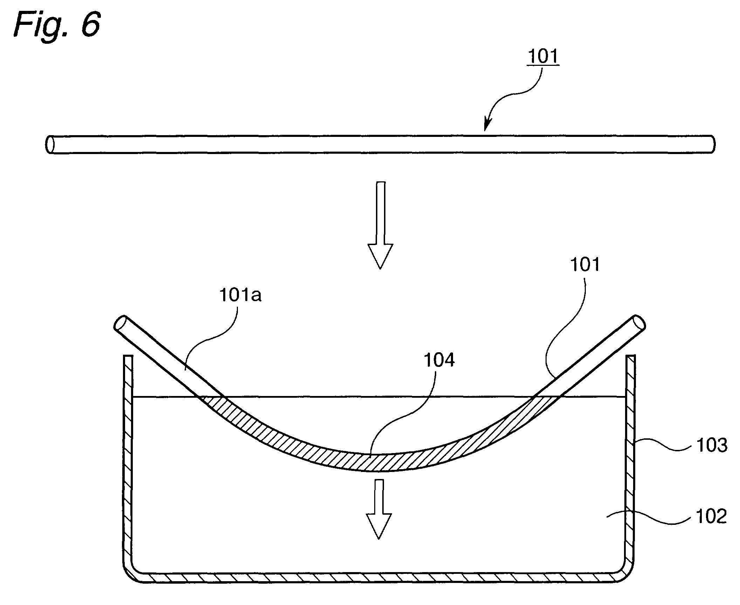

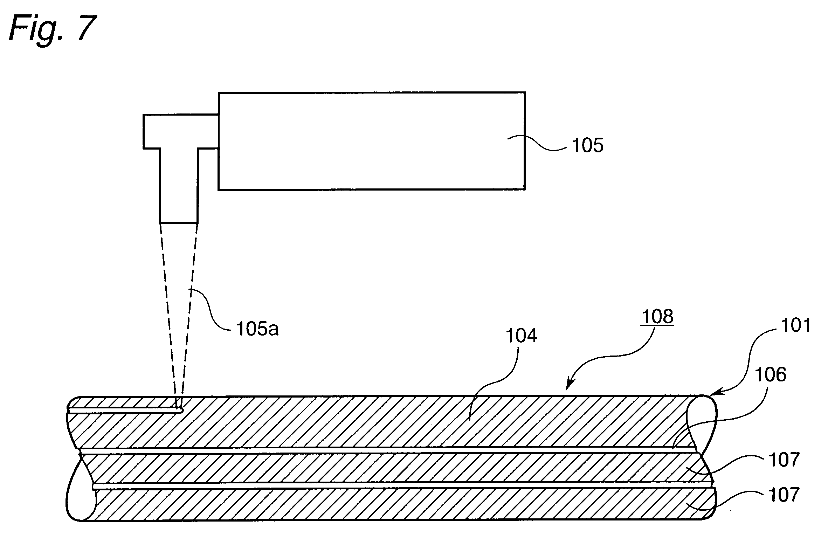

Fig. 6 is a schematic diagram explaining one mode of step of coating with a conductive solution, conducted in the process for producing a lead wire according to the present invention. Fig. 7 is a schematic diagram explaining one mode of step of forming insulating zones by irradiation working, conducted in the process for producing a lead wire according to the present invention.

Referring to Fig. 6, numeral 101 denotes a lead wire substrate which constitutes the body of the lead wire of the present invention. The lead wire substrate 101 has the shape of a tube and is composed of, for example, a polyolefin resin such as polyethylene or polypropylene, a polyurethane resin, a polyester resin or a polycarbonate resin. The lead wire substrate 101 may be composed of any highly flexible polymeric materials other than these, and the materials are not particularly limited. Although the size of the lead wire substrate 101 can also be appropriately changed according to the purpose of the use, it is preferably in the range of, for example, about 0.1 to 20 mm in outside diameter, 0.003 to 5 mm in thickness and 500 to 2000 mm in length in the use in, for example, the medical field. The lead wire substrate 101 of the above construction is dipped in dip coating bath 103 in which conductive solution 102 is placed. Thereafter, the lead wire substrate 101 is taken out from the dip coating bath 103 and dried, so that conductive thin-film layer 104 is formed on the outer wall 101a of the lead wire substrate 101.

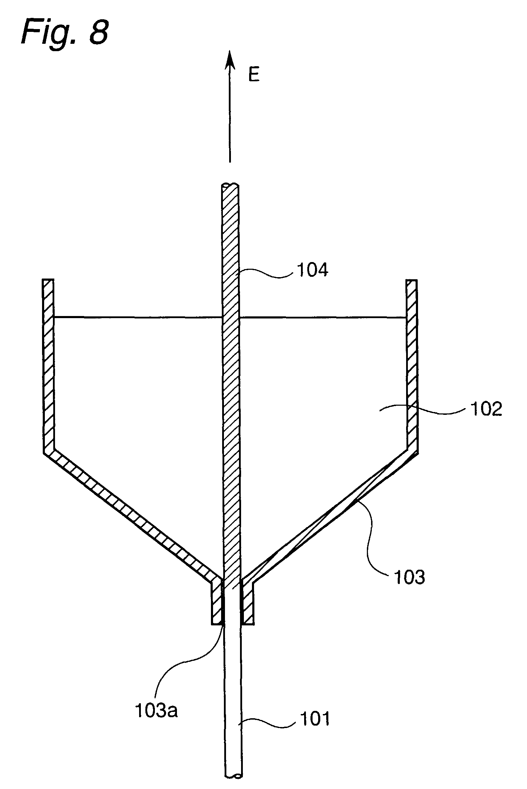

In place of the above dipping of the lead wire substrate 101 in the dip coating bath 103 to thereby form the conductive thin-film layer 104 on the outer wall 101a of the lead wire substrate 101, as shown in Fig. 8, the lead wire substrate 101 can be passed through hole 103a provided at the bottom of the dip coating bath 103 and pulled upward in the arrowed E direction, so that the conductive thin-film layer 104 can be continuously formed on the outer wall 101a of the lead wire substrate 101.

The paste material constituting the principal agent of the conductive solution 102 is preferably selected from among, for example, epoxy, polyimide, polyester, acrylic, vinyl and phenol resins.

The conductive material as a constituent of the conductive solution is, for example, a conductive metal such as Au, Ag, Cu, Al or Pt, or a nonmetallic conductive material such as conductive carbon. These materials are used either individually or in combination.

Referring to Fig. 7, the lead wire substrate 101 having its outer surface furnished with the conductive thin-film layer 104 is irradiated with electron beam or light 105a from irradiation working apparatus 105 on the surface thereof and along the lead wire. Thus, the conductive thin-film layer 104, at the irradiated parts, is removed, so that channel-shaped insulating zones 106 are formed.

The irradiation working apparatus 105 is numerically controlled by a computer. A plurality of insulating zones 106 are formed by repeated scanning along the direction of the length of the lead wire substrate 101. As a result, the conductive thin-film layer 104 remaining on the surface of the lead wire substrate 101 forms a plurality of conductive lines 107 electrically insulated from each other, which border the insulating zones 106 and are arranged along the lead wire 108.

The light emitted from the irradiation working apparatus 105 is preferably laser beams, such as excimer, YAG or carbon dioxide laser beams, having a wavelength of 0.19 to 10.6 µm. From the viewpoint of working capability, it is especially preferred that excimer laser beams having a wavelength of 0.19 to 0.35 µm is used.

Although the width, depth, etc. of the insulating zones 106 can be appropriately selected in conformity with the dimension of the lead wire substrate 101, it is preferred that the width range from 10 to 60 µm from the viewpoint that short circuiting can be prevented and insulating capability is ensured. Further, although the insulating zones 106 are formed along the lead wire substrate 101 in the direction of the length thereof as shown in Fig. 7, the configuration of the insulating zones 106 can be appropriately changed, for example, can have spiral form (not shown).

In this irradiation working, unless the lead wire substrate 101 is irradiated with the laser or the like emitted from the irradiation working apparatus 105 while the position thereof is accurately fixed, there is the danger of, for example, short circuiting of the conductive lines 107 constituting electric conduction paths.

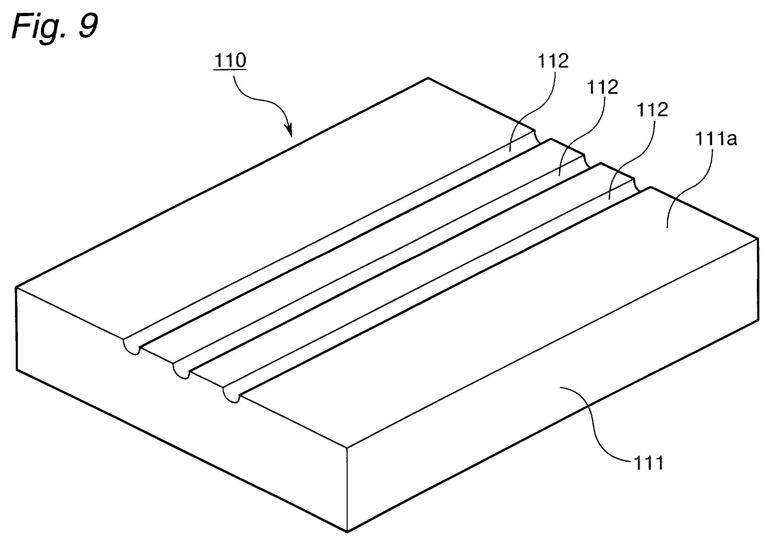

Therefore, irradiation working jig 110 as shown in Fig. 9 is preferably used in the irradiation working. The irradiation working jig 110 comprises base frame 111 having the shape of a plate. The base frame 111 at its upper surface 111a is provided with a plurality of fixing channels 112, 112 having a semicircular section, which are arranged with a constant spacing along the length of the base frame 111 and which are used to fix the lead wire substrate 101. The dimension of the fixing channels is slightly smaller than or identical with that (diameter) of the lead wire substrate 101, so that the lead wire substrate 101 can be unmovably fixed in the fixing channels 112.

By virtue of the above fixing of the lead wire substrate 101 in the fixing channels 112 of the base frame 111, the dislocation of the lead wire substrate 101 can be avoided at the time of irradiation with, for example, laser emitted from the irradiation working apparatus 105. Therefore, not only the insulating zones 106 can be accurately formed but also the conductive lines 107 constituting electric conduction paths are not dislocated and short circuiting thereof does not occur. Moreover, a plurality of fixing channels 112, 112 are provided, so that a plurality of lead wire substrates 101 can be simultaneously fixed and subjected to irradiation working to thereby enhance the productivity thereof.

In the use of this working jig 110, it is required for forming a plurality of insulating zones 106 that, after completing irradiation working once, the lead wire substrate 101 should be reset in the fixing channel 112 in order to change the position of irradiation working.

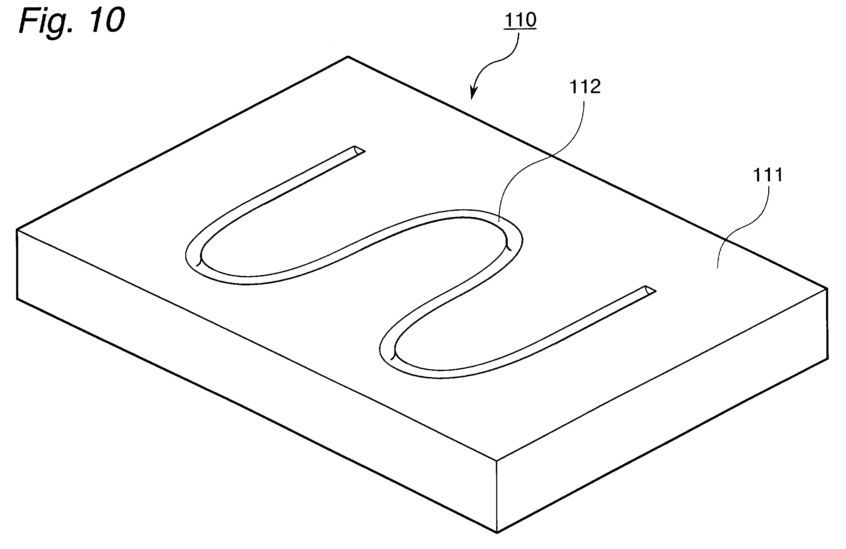

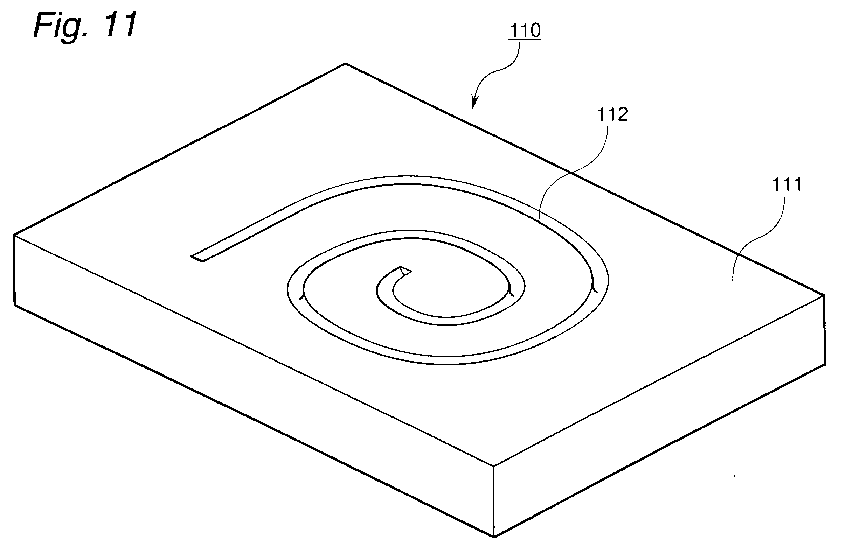

Although in this embodiment the fixing channel 112 is provided linearly along the direction of the length of the base frame 111, it is naturally feasible to arrange the fixing channel 112 in curved form as shown in Figs. 10 and 11. In this instance, the irradiation working apparatus 105 is numerically controlled by a computer so that, in conformity with the configuration of the lead wire substrate 101 fixed in the fixing channel 112 of the base frame 111, the irradiation working can be performed by scanning the surface of the lead wire substrate 101 along the lead wire substrate 101 on the basis of the data inputted in advance. The employment of this arrangement enables irradiation working without the need to enlarge the dimension of the base frame 111, namely, the dimension of the working jig 110 even when the length of the lead wire is large.

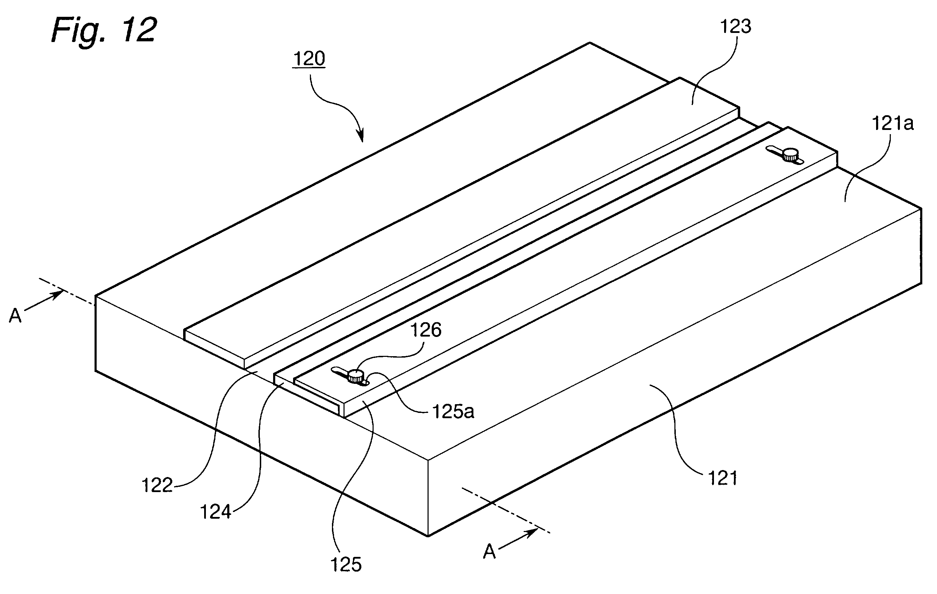

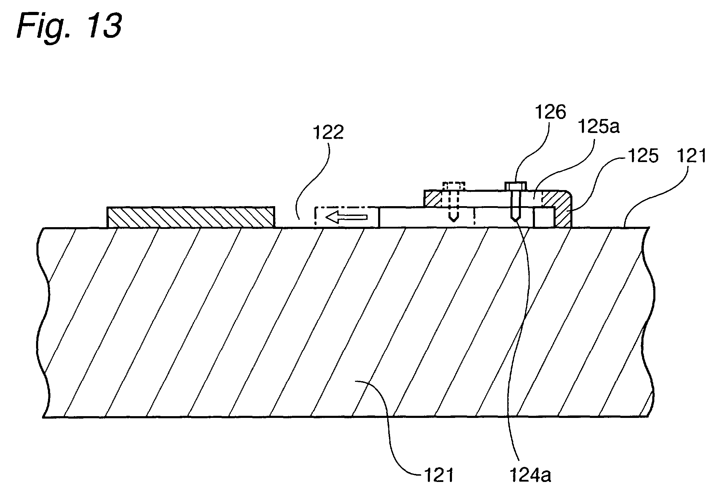

Fig. 12 is a perspective view of a further form of working jig according to the present invention, and Fig. 13 is a sectional view on the line A-A of Fig. 12.

In working jig 120 of this embodiment, fixed plate 123 having the shape of a band plate is fixed on upper surface 121a of base frame 121 having the shape of a plate in the direction of the length of the working jig 120. Accommodating part 125 having an L-shaped section which accommodates movable plate 124 having the shape of a band plate in a manner such that the movable plate 124 can slide toward the fixed plate 123 and backward is fastened on the upper surface 121a of the base frame 121 with a given spacing from the fixed plate 123.

Width regulating screw 126 is fitted in threaded hole 124a formed in the upper surface portion of the movable plate 124 through slit 125a formed in the upper surface portion of the accommodating part 125. The movable plate 124 is slid away from the fixed plate 123 by loosening the width regulating screw 126 to thereby provide fixing channel 122 between the movable plate 124 and the fixed plate 123. The lead wire substrate 101 is fitted in the fixing channel 122, and, thereafter, the movable plate 124 is slid toward the fixed plate 123. The lead wire substrate 101 can thus be fixed in the fixing channel 122 by driving the width regulating screw 126.

This arrangement enables changing the width of the fixing channel 122 by sliding the movable plate 124 toward the fixed plate 123 and backward, so that it is feasible to cope with various dimensions of the lead wire substrate 101.

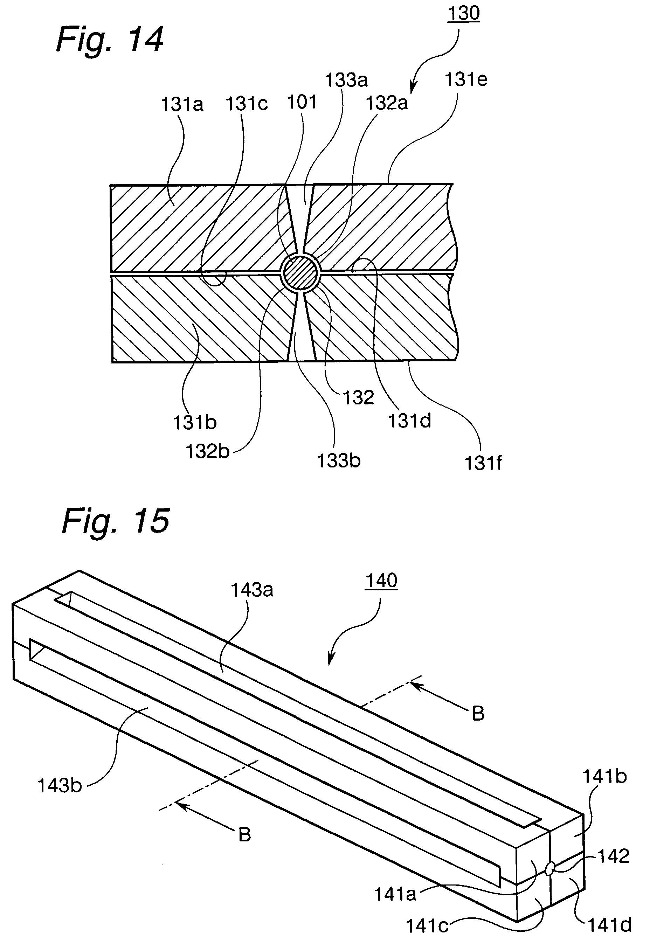

Fig. 14 is a partially enlarged sectional view of still a further form of working jig according to the present invention.

Working jig 130 of this embodiment comprises a pair of base frames consisting of upper base frame 131a and lower base frame 131b. These base frames 131a, 131b have respective matching faces 131c, 131d which are provided with respective fixing channels 132a, 132b having an approximately semicircular section. Space 132 is formed between the fixing channels 132a, 132b by closing the base frames 131a, 131b, and the lead wire substrate 101 is fixed in the space 132.

Irradiation slits 133a, 133b are respectively formed in the upside base frame 131a, from back 131e to the fixing channel 132a, and in the downside base frame 131b, from back 131f to the fixing channel 132b. Arrangement is so made that the irradiation working of the surface of the lead wire substrate 101 is conducted through the irradiation slits 133a, 133b. This arrangement enables accurately forming two insulating zones 106 on the upper side and lower side of the lead wire substrate 101, respectively, at one time.

In this instance, it is naturally feasible to provide the fixing channel 132 linearly along the direction of the length of the base frame 131 as in the embodiment of Fig. 9 and also to arrange the fixing channel 132 in curved form as in the embodiments of Figs. 10 and 11.

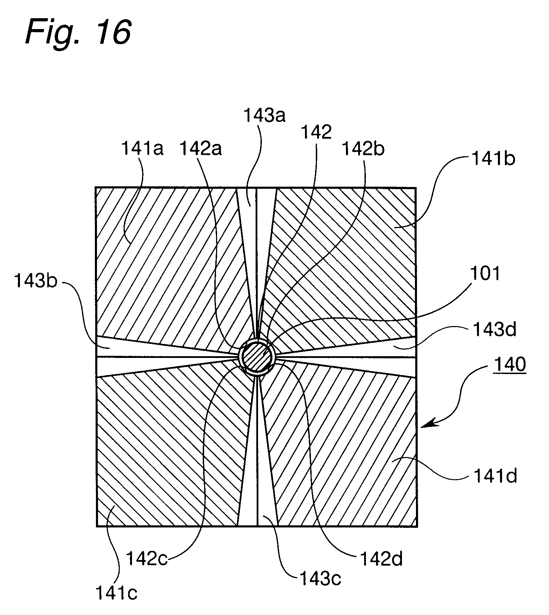

Fig. 15 is a perspective view of still a further form of working jig according to the present invention, and Fig. 16 is a sectional view on the line B-B of Fig. 15. Working jig 140 of this embodiment has the shape of substantially a square pole and consists of base frames 141a to 141d which correspond to four divisions of the square pole, namely, upper left, upper right, lower left and lower right parts of the square pole. The matching faces of these base frames are provided with fixing channels 142a to 142d having an approximately quadrantal section. Space 142 is defined between the fixing channels 142a to 142d by closing the base frames 141a to 141d together, and the lead wire substrate 101 is fixed in the space 142.

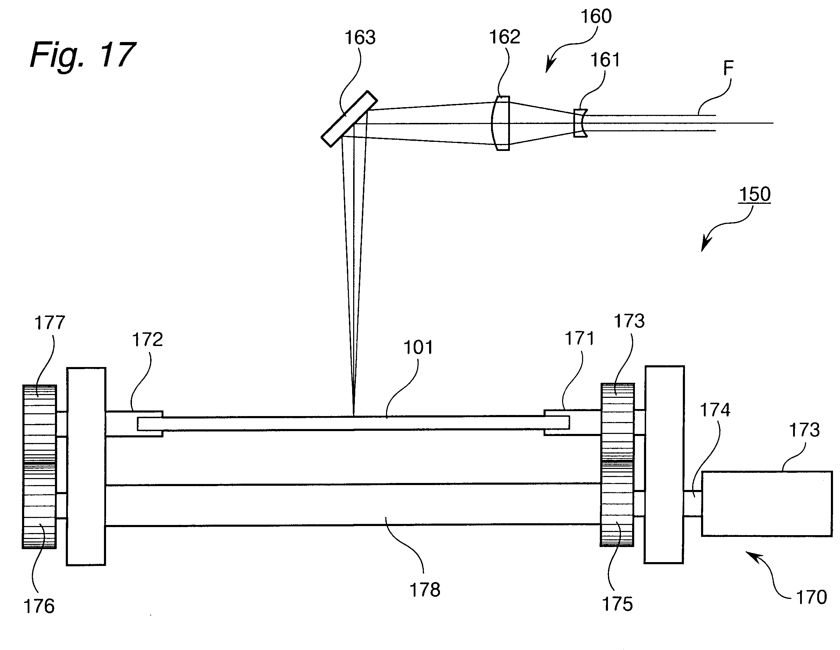

The base frames 141a to 141d are provided with four-direction irradiation slits 143a to 143d which, when the base frames 141a to 141d are closed together, are directed from the space 142 defined by the fixing channels 142a to 142d upward, leftward, downward and rightward, respectively. Arrangement is so made that the irradiation working of the surface of the lead wire substrate 101 with the use of the irradiation working apparatus is conducted through the four-direction irradiation slits 143a to 143d. This arrangement enables accurately forming four insulating zones 106 on the upper side, left side, lower side and right side of the lead wire substrate 101, respectively, at one time. Fig. 17 is a schematic diagram of another form of irradiation working apparatus for use in the present invention.

This form of irradiation working apparatus 150 comprises laser irradiation apparatus 160 capable of laser irradiation and scanning and irradiation working jig 170 disposed thereunder.

Input laser beam F generated by a laser source (not shown) is transmitted through lenses 161, 162 and reflected by scanner 163 provided with a mirror, and the lead wire substrate 101 fixed in the irradiation working jig 170 disposed thereunder is irradiated with the laser beam F.

On the other hand, the irradiation working jig 170 is fitted with clamps 171, 172 for fixing both ends of the lead wire substrate. The clamp 171 is fastened to driving shaft 174 of stepping motor 173. Gear 173 fastened to the driving shaft 174 engages clamp timing gear 175 fastened to one end of synchronous shaft 178. Revolution of the driving shaft 174 is transmitted to the clamp 172 through gear 177 which engages clamp timing gear 176 fastened to the other end of the synchronous shaft 178. Therefore, the clamps 171, 172 have mutually synchronous revolution in accordance with the stepwise revolution of the driving shaft 174 effected by the stepping motor 173.

The method of forming insulating zones 106 on the surface of the lead wire substrate 101 by the use of the irradiation working apparatus 150 of the above construction is as follows.

First, both ends of the lead wire substrate 101 are fixed with a tension on the clamps 171, 172. Then, the laser irradiation apparatus 160 is operated. Input laser beam F generated by a laser source is transmitted through lenses 161, 162 and reflected by scanner 163 fitted with a mirror, and the lead wire substrate 101 fixed on the clamps 171, 172 of the irradiation working jig 170 is irradiated with the laser beam F. The irradiation is moved in the direction of the length of the lead wire substrate 101, so that a channel is formed along the direction of the length of the lead wire substrate 101. Thus, the insulating zone 106 is obtained.

After the formation of the insulating zone 106 effected by the channeling along the direction of the length of the lead wire substrate 101, the stepping motor 173 of the irradiation working jig 170 is driven so that the driving shaft 174 is 90° stepwise revolved. Synchronous revolution of the clamps 171, 172 is performed so that the lead wire substrate 101 is 90° revolved.

Thereafter, the laser irradiation apparatus 160 is operated so that the lead wire substrate 101 is irradiated with laser beam F. This cycle is repeated four times, thereby enabling accurately forming four insulating zones 106 on the, upper, left, lower and right parts of each lead wire substrate 101, respectively.

The number of insulating zones 106 formed on the periphery of the lead wire substrate 101 can be changed by varying the angle of revolution of the stepping motor 173 in place of the 90° stepwise revolution conducted in this embodiment.

Although in the above embodiments the outer wall 101a of the lead wire substrate 101 is coated with the conductive solution 102 by subjecting the lead wire substrate 101 to the dip coating to thereby enable forming the conductive thin-film layer 104, it is feasible to coat the outer wall 101a of the lead wire substrate 101 with the conductive solution 102 by other methods such as the roll coating.

Although not shown, the formation of insulating zones 106 and, hence, conductive lines 107 by the irradiation working of the lead wire substrate 101 can naturally be followed by coating of the surface of the lead wire 108 with a highly flexible polymeric material such as a silicone resin.

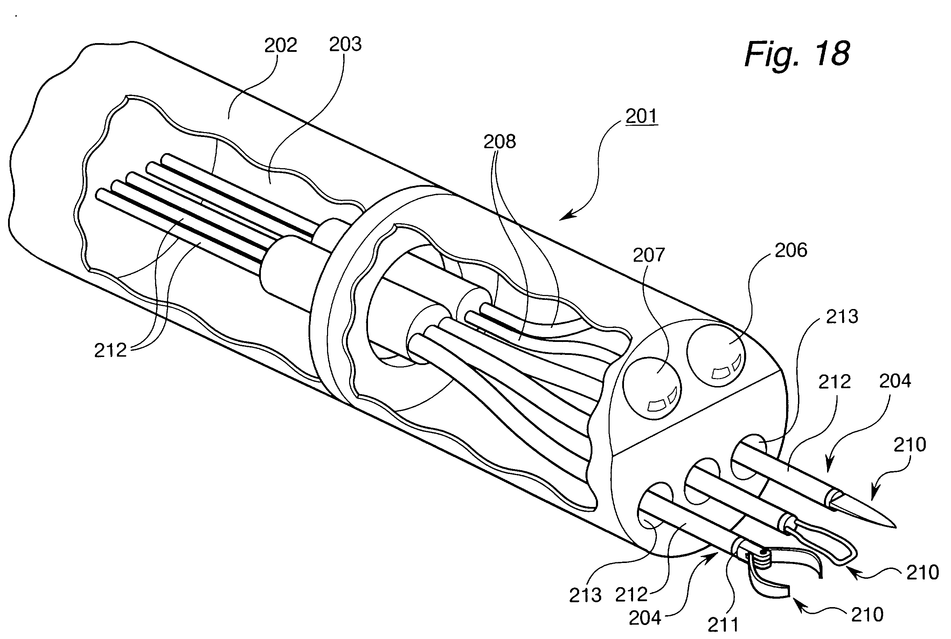

Fig. 18 is a partially cutaway perspective view of the first form of micromachine fitted with the microdevice of the present invention applied to the medical field, especially, microsurgery field. Referring to Fig. 18, numeral 201 generally denotes a micromachine. The micromachine 201 comprises tubular endoscope body 202. The inside of the endoscope body 202 is provided with internal spaces for microdevice 203 which are formed in the direction of the length of the endoscope body 202. A plurality of microdevices 204 are fitted in the internal spaces for microdevice 203. Front end portion 205 of the endoscope body 202 is equipped with irradiation lens 206 and photographic lens 207. These lenses are connected, through optical fiber 208 disposed in the internal spaces for microdevice 203 provided inside the endoscope body 202, to a control unit not shown.

On the other hand, referring to Fig. 18, the microdevice 204 comprises slender guide member body 212 and, connected to an end thereof through connecting part 211, therapeutic tool 210 such as biopsy forceps, other forceps, scissors, a snare or a cell harvesting brush. Arrangement is so made that the therapeutic tools 210 and the vicinities of the front ends of the guide member bodies 212 thereof protrude from openings for microdevice 213 provided at the front end of the endoscope body 202.

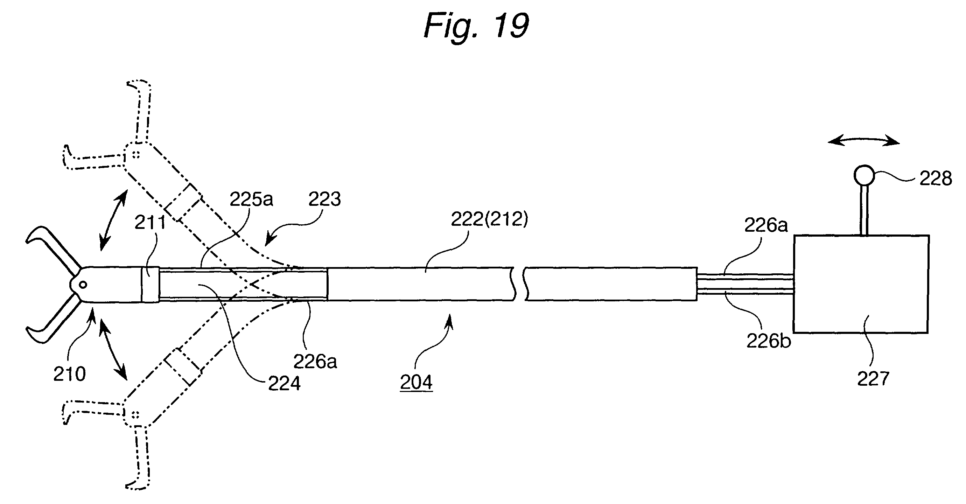

Referring now to Fig. 19, the microdevice 204 is provided with linear member 222 which consists of a slender tube of, for example, a synthetic resin or stainless steel and which is a constituent of the guide member 212 and polymeric actuator 223 joining thereto at the vicinity of the connecting part 211 of the guide member 212.

This polymeric actuator 223 comprises ion exchange resin molding 224 shaped into a relatively slender rectangular plate and, adhering to both sides thereof, at least a pair of electrodes 225a, 225b. Application of a voltage to the pair of electrodes 225a, 225b causes the ion exchange resin molding 224 to undergo curving deformation (bending) in two directions.

Ends of a pair of lead wires 226a, 226b are electrically connected to the electrodes 225a, 225b, respectively. The lead wires 226a, 226b are positioned inside the linear member 222 and extend along the entire length of the linear member 222, and the other ends of the lead wires 226a, 226b are connected to operation control unit 227.

This operation control unit 227 is fitted with operating lever 228 which enables switching operation. The electrode of the current from power source 230 which flows through the pair of lead wires 226a, 226b can be switched by means of double-pole double-throw switch 229 disposed inside the operation control unit 227 in accordance with the manipulation made by the operating lever 228.

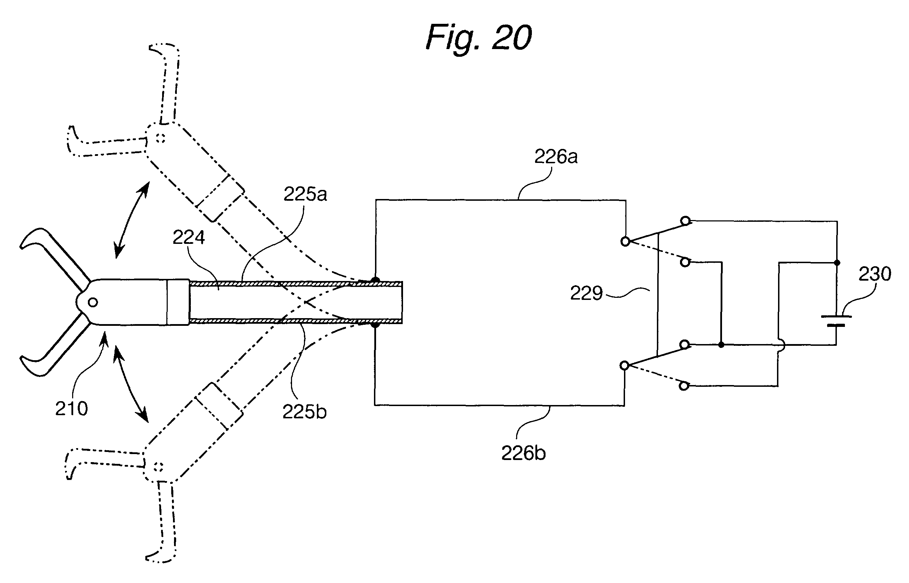

That is, referring to Fig. 20, when the doublepole double-throw switch 229 is arranged in the position indicated by the full line, the lead wires 226a and 226b are connected to the positive electrode and the negative electrode, respectively. On the other hand, when the double-pole double-throw switch 229 is rearranged from the neutral position as indicated by the two-dot long and two short dashes line in accordance with the manipulation made by the operating lever 228 of the operation control unit 227, contrarily, the lead wires 226a and 226b are connected to the negative electrode and the positive electrode, respectively.

Although in this embodiment the switching is performed by the use of the double-pole double-throw switch 229, this is no limitation and any common electrical circuits for electrode change-over can be used.

This actuator 223 has been developed as an actuator element which can easily be miniaturized, ensures rapid response and operates with low electric power (see Japanese Patent Laid-open Publication No. 4(1992)-275078). Both a cation exchange resin membrane and an anion exchange resin membrane can be used as the ion exchange resin molding 224 for forming the polymeric actuator 223. For example, the cation exchange resin membrane can be a polystyrenesulfonate membrane or an ion exchange resin membrane of a fluororesin having, introduced therein, a sulfonate group or a carboxyl group. Furthermore, the actuator element produced by the above process for producing an actuator element according to the present invention can be also used.

Although a noble metal such as gold, platinum, iridium, palladium or ruthenium is preferred, other materials having both conductivity and corrosion resistance, such as a conductive polymer or graphite, can also be used in the electrodes 225a, 225b joined to both the surfaces of the ion exchange resin molding 224. The joining of the electrodes 225a, 225b to the ion exchange resin molding 224 can be performed by the use of the known method for bonding an electrode material to a polymeric membrane, such as chemical plating, electroplating, vacuum deposition, sputtering, coating, press bonding or fusion bonding. The connection of the lead wires 226a, 226b to the electrodes 225a, 225b can be performed by the common method, such as the application of a conductive adhesive, metal welding, fitting, caulking or application of another adhesive. The material for forming the lead wires 226a, 226b can be selected from among common conductive materials such as copper, gold, silver, iron and aluminum, which, according to necessity, can be plated or provided with an insulating coating. Further, it is feasible to arrange the lead wires 226a, 226b outside the linear member 222 or to print a conductive ink on the inner wall surface or outer wall surface of the linear member 222 so that it is used as a lead wire. Still further, use can also naturally be made of the lead wire produced by the above process for producing a lead wire according to the present invention.

In this embodiment, a fluororesin ion exchange membrane having a thickness of 100 to 200 µm, a width of 300 µm and a length of 14 mm is used as the ion exchange resin molding 224. Both the surfaces thereof are chemically plated with gold. This gold is used as the electrodes 225a, 225b. Thus, the polymeric actuator 223 is formed. A 1 to 3 V direct current power source is used as the power source 230.

Although the mechanism of operation of this polymeric actuator 223 is not necessarily apparent, it is presumed that, by the application of a potential difference between the front and back surfaces of the ion exchange resin molding 224, positive ions lying in the ion exchange resin molding 224 move to the negative electrode side and, entrained by the ions, water molecules move in the ion exchange resin molding 224. As a result, a water content difference occurs between the positive electrode side and the negative electrode side. Thus, it is presumed that the increase in water content causes a swelling and the decrease in water content causes a shrinkage, so that the ion exchange resin molding 224 is curved.

That is, when the lead wires 226a and 226b are simultaneously connected to the positive (+) side and the negative (-) side of the power source 230, respectively, as shown by the full line in Fig. 20 by means of the double-pole double-throw switch 229 by manipulating the operating lever 228 of the operation control unit 227, the electrode 225a connected to the lead wire 226a becomes a positive electrode while the electrode 225b connected to the other lead wire 226b becomes a negative electrode. As a result, the ion exchange resin molding 224 is curved from the linear neutral position shown by the full line in Fig. 20 upward as shown by the dotted line in Fig. 20.

On the other hand, when the lead wires 226a and 226b are simultaneously connected to the negative (-) side and the positive (+) side of the power source 230, respectively, as shown by the two-dot long and two short dashes line in Fig. 20 by manipulating the operating lever 228 of the operation control unit 227, the electrode 225a connected to the lead wire 226a becomes a negative electrode while the electrode 225b connected to the other lead wire 226b becomes a positive electrode. As a result, the ion exchange resin molding 224 is curved downward as shown by the two-dot long and two short dashes line in Fig. 20.

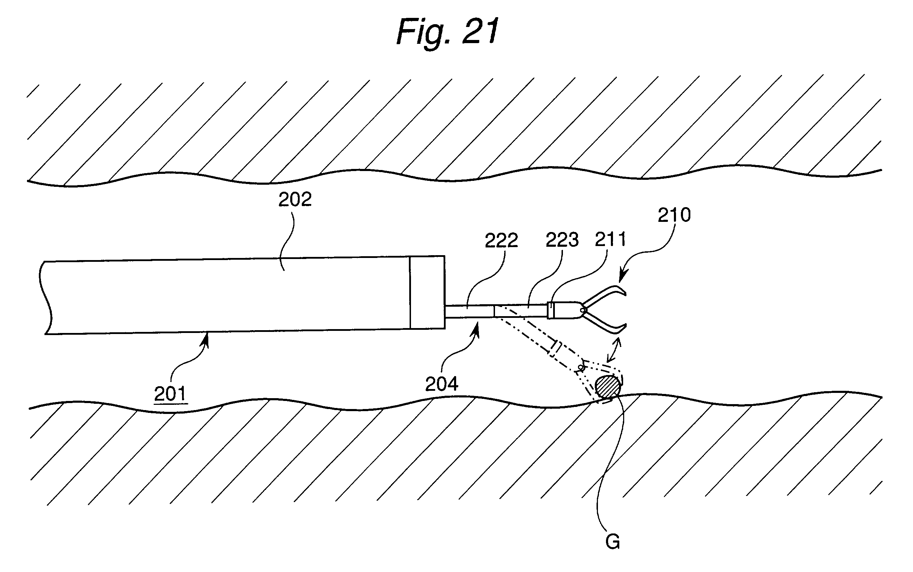

Thus, the polymeric actuator 223 joined to the front end of the linear member 222, namely, to the guide member 212 of the microdevice 204 at the vicinity of the connecting part 211 can be arbitrarily and positively deformed by manipulating the operating lever 228 of the operation control unit 227, disposed at the handy side. Therefore, by virtue of the free deformation of the polymeric actuator 223, the therapeutic tool 210, such as biopsy forceps, other forceps, scissors, a snare, a cell harvesting brush or a therapeutic knife, connected to the front end of the guide member 212 can arbitrarily be guided toward, for example, lesion G inside an organ, as shown in Fig. 21, to thereby enable resecting or extirpating the lesion.

In this embodiment, the outside diameter of the linear member 222 is preferably up to 5 mm, still preferably, up to 1 mm in connection with the inside diameter of the endoscope body 202. The voltage applied to the electrodes 225a, 225b is, for example, up to 10 V. The voltage is preferred to be about 2 V when a gas is emitted depending on the type of the material of the electrode.

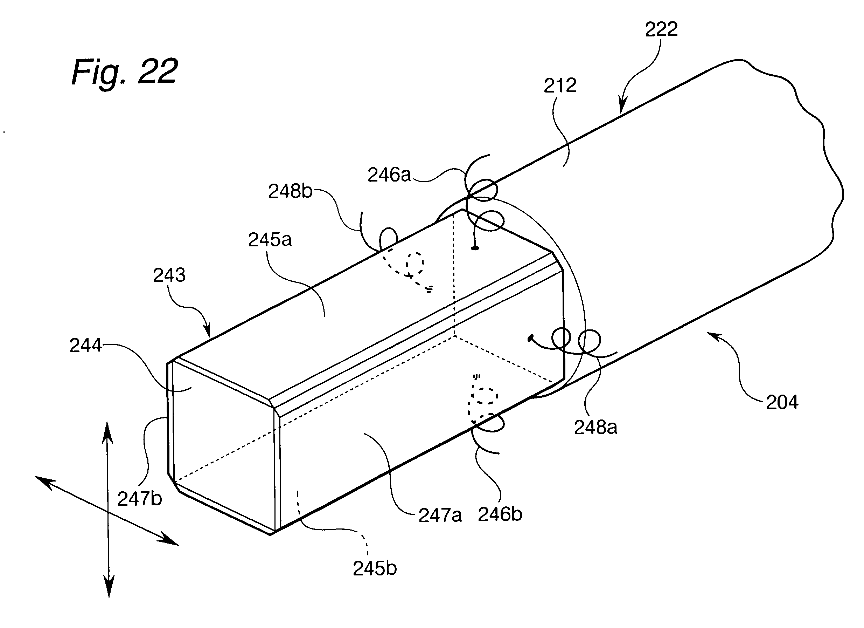

Fig. 22 shows another form of microdevice of the present invention, which corresponds to the first form of microdevice having polymeric actuator 243, which is shaped into a square pole, joined to the front end of the linear member 222. Therapeutic tool 210 is not shown in the figure for the convenience of elucidation (same in the following embodiments).

Specifically, the polymeric actuator 243 comprises ion exchange resin molding 244 having the shape of a square pole of, for example, 300 to 600 µm square and 14 mm length and two pairs (four) of electrodes 245a, 245b, 247a, 247b disposed in mutually electrically insulated relationship on the four sides of the ion exchange resin molding 244, respectively. Ends of lead wires 246a, 246b, 248a, 248b composed of, for example, a 30 µm iron wire provided with an insulating coating are electrically connected by means of, for example, tin solder to the above electrodes 245a, 245b, 247a, 247b, respectively. Application of a voltage to the electrodes 245a, 245b, 247a, 247b arranged opposite to each other with the ion exchange resin molding 244 interposed therebetween realizes a four-direction curving.

In this embodiment, the above electrodes 245a, 245b, 247a, 247b are formed by uniformly applying, for example, a gold plating to the four sides, except the two end faces, of the ion exchange resin molding 244 and, thereafter, removing the gold plating at the four corners of the ion exchange resin molding 244 along the direction of the length thereof.

In this embodiment, the polymeric actuator 243 is furnished with the two pairs of electrodes 245a, 245b, 247a, 247b arranged opposite to each other with the ion exchange resin molding 244 interposed therebetween, and a four-direction arbitrary curving of the polymeric actuator 243 can be realized by applying a voltage to these electrodes 245a, 245b, 247a, 247b, as in the foregoing embodiments. Further, the curving directions can be combined with each other to thereby enable a rotation.

Although an arrangement can be made so as to effect the rotation by the use of three electrodes, the rotation can be performed more smoothly by the use of four (two pairs of) electrodes.

In this embodiment, although the lead wires 246a, 246b, 248a, 248b are positioned inside the linear member 222, extend along the length of the linear member 222 and reach the operation control unit 227 as in the embodiment of Fig. 18, they may be arranged outside the linear member 222.

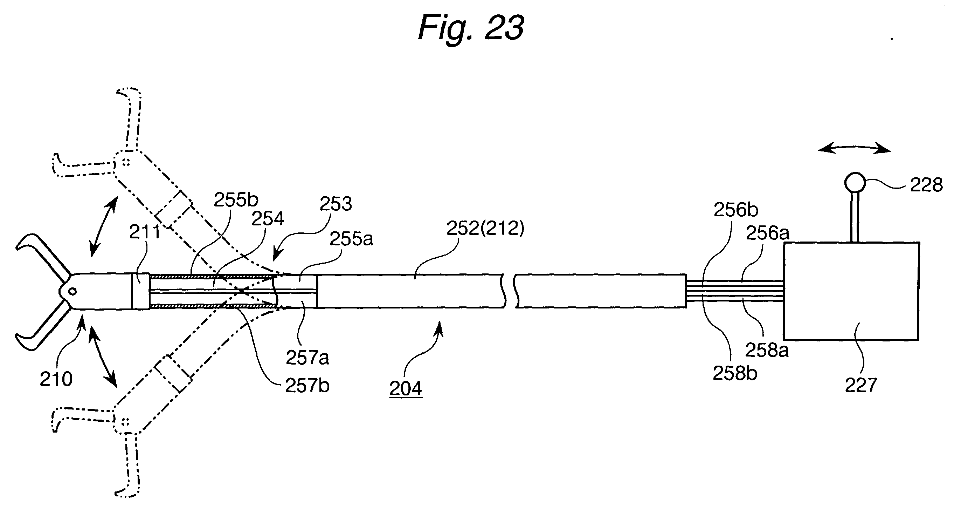

Fig. 23 shows still another form of microdevice of the present invention. In this embodiment, the guide member 212 comprises tube body 252 composed of, for example, a synthetic resin or stainless steel and tubular polymeric actuator 253 arranged at curved part, which constitutes connecting part 211, of the front end of the tube body 252.

That is, microdevice in which, as in the above embodiments, use is made of the polymeric actuator having a rectangular shape or the shape of a square pole and in which the guide member 212 consists of linear member 222 is available when the therapeutic tool 210 is a therapeutic knife or a cell harvesting brush. However, when the therapeutic tool 210 is one which must be capable of performing closing and opening operation, such as biopsy forceps, other forceps, scissors or a snare, it is needed to arrange an operating wire or the like inside the linear member 222 but it is difficult to meet the need. In contrast, the employment of the guide member 212 and polymeric actuator 253 having tubular shapes as in this embodiment facilitates the arranging of the operating wire (not shown) inside the guide member 212.

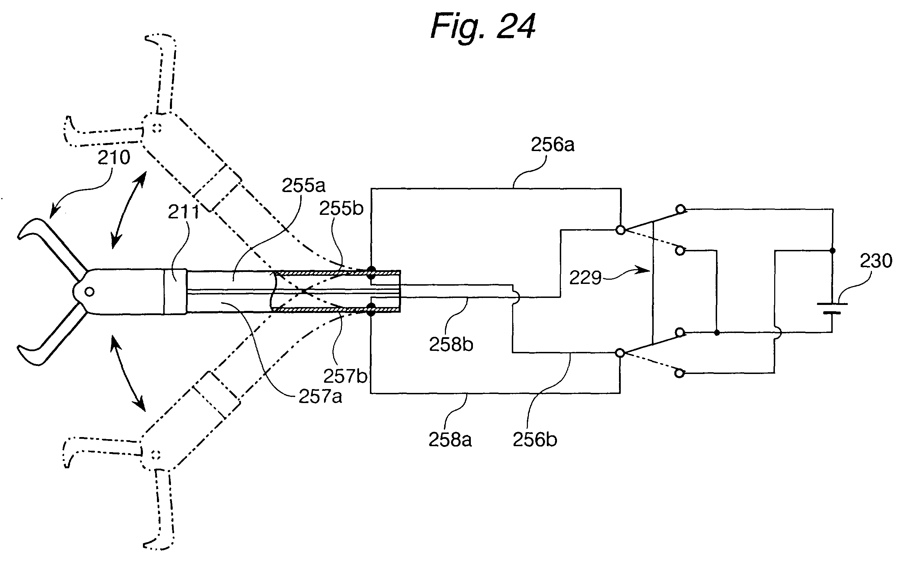

The polymeric actuator 253 comprises cylindrical ion exchange resin molding 254 and two pairs (four) of electrodes 255a, 255b, 257a, 257b disposed in mutually electrically insulated relationship on inner wall surface and outer wall surface positions, opposite to each other, of the ion exchange resin molding 254 with the ion exchange resin molding 254 interposed therebetween. Application of a voltage to each of the two pairs of electrodes 255a, 255b, 257a, 257b realizes a two-direction curving deformation (bending) of the ion exchange resin molding 254. Ends of lead wires 256a, 256b, 258a, 258b are electrically connected to the electrodes 255a, 255b, 257a, 257b, respectively. The lead wires 256a, 256b, 258a, 258b are positioned inside the tube body 252 and extend along the entire length of the tube body 252, and the other ends of the lead wires 256a, 256b, 258a, 258b are connected to the operation control unit 227, as in the first embodiment of Fig. 19. That is, referring to Fig. 24, when the doublepole double-throw switch 229 is arranged in the position indicated by the full line, the lead wires 256a and 258b are connected to the positive (+) electrode and the lead wires 256b and 258a to the negative (-) electrode. On the other hand, when the double-pole double-throw switch 229 is rearranged from the neutral position as indicated by the two-dot long and two short dashes line in accordance with the manipulation made by the operating lever 228 of the operation control unit 227, contrarily, the lead wires 256a and 258b are connected to the negative (-) electrode and the other lead wires 256b and 258a to the positive (+) electrode.

That is, when the lead wires 256a and 258b are connected to the positive (+) side of the power source 230 and the lead wires 256b and 258a simultaneously to the negative (-) side of the power source 230 as shown by the full line in Fig. 24 by means of the double-pole double-throw switch 229 by manipulating the operating lever 228 of the operation control unit 227, the electrodes 255a and 257b connected to the lead wires 256a and 258b become positive electrodes while the electrodes 255b and 257a connected to the lead wires 256b and 258a become negative electrodes. As a result, the ion exchange resin molding 254 is curved from the linear neutral position shown by the full line in Fig. 24 upward as shown by the dotted line in Fig. 24.

On the other hand, when the lead wires 256a and 258b are connected to the negative (-) side of the power source 230 and the lead wires 256b and 258a simultaneously to the positive (+) side of the power source 230 as shown by the two-dot long and two short dashes line in Fig. 24 by manipulating the operating lever 228 of the operation control unit 227, the electrodes 255a and 257b connected to the lead wires 256a and 258b become negative electrodes while the electrodes 255b and 257a connected to the lead wires 256b and 258a become positive electrodes. As a result, the ion exchange resin molding 254 is curved downward as shown by the two-dot long and two short dashes line in Fig. 24.

The reason for the above connection of the upper lead wires and the lower lead wires, among the pairs of lead wires 256a, 256b, 258a, 258b, to respective single electrodes is to avoid the mutual cancellation of curving directions as the ion exchange resin molding 254 is so curved that the positive electrode side shrinks as mentioned below.

In this embodiment, for example, a fluorinated ion exchange resin having an inside diameter of 600 µm, an outside diameter of 800 µm and a length of 15 mm is used as the ion exchange resin molding 254. Both the inner wall surface and outer wall surface thereof are uniformly chemically plated with gold. Further, insulating zones 259 composed of a part having gold plating removed are provided at two positions of each of the inner wall surface and outer wall surface along the direction of the length of the ion exchange resin molding 254, which halve the ion exchange resin molding 254. Thus, the polymeric actuator 253 fitted with electrodes 255a, 255b, 257a, 257b of gold plating is formed. A 1 to 3 V direct current power source is used as the power source 230.

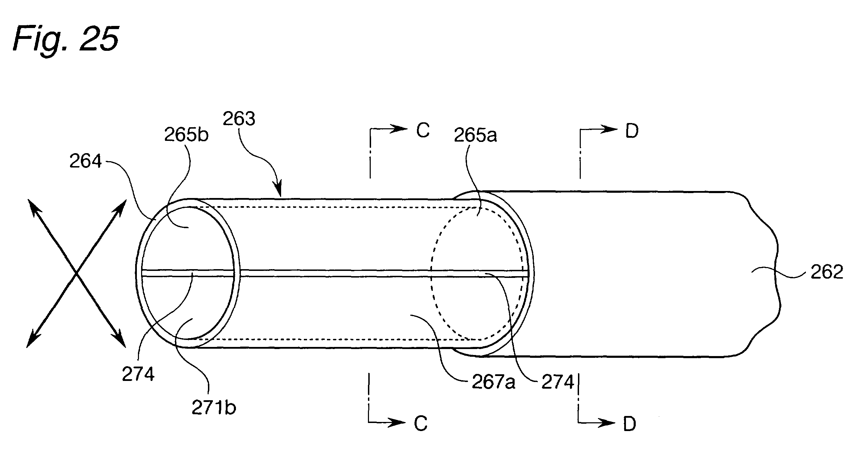

Figs. 25 to 27 show a further form of microdevice of the present invention. In this embodiment, the guide member 212 comprises a double tube, as tube body 262, consisting of inside tube 262a and outside tube 262b and, joined to a front end of the tube body 262, cylindrical polymeric actuator 263.

Specifically, the polymeric actuator 263 comprises cylindrical ion exchange resin molding 264 having, for example, an inside diameter of 600 µm, an outside diameter of 800 µm and a length of 15 mm and four pairs (eight) of electrodes 265a, 265b, 267a, 267b, 269a, 269b, 271a, 271b disposed in mutually opposite positions of the inner wall surface and outer wall surface of the ion exchange resin molding 264. Ends of lead wires 266a, 266b, 268a, 268b, 272a, 272b, 274a, 274b composed of, for example, a 30 µm iron wire provided with an insulating coating are electrically connected by means of, for example, tin solder to the above electrodes 265a, 265b, 267a, 267b, 269a, 269b, 271a, 271b, respectively. Application of a voltage to the electrodes 265a, 265b, 267a, 267b, 269a, 269b, 271a, 271b arranged opposite to each other with the ion exchange resin molding 264 interposed therebetween realizes a four-direction curving.

In this construction, for avoiding the mutual cancellation of curving directions, voltage must be applied so that moving directions are identical with each other between the electrodes positioned on mutually opposite sides, namely, between the electrodes 265a, 265b and the electrodes 269a, 269b and between the electrodes 267a, 267b and the electrodes 271a, 271b. The above electrodes 265a, 265b, 267a, 267b, 269a, 269b, 271a, 271b are formed by uniformly applying a gold plating to the outer wall surface and inner wall surface of the ion exchange resin molding 264 and, thereafter, providing insulating zones 274 composed of a part having gold plating removed at four positions of each of the outer wall surface and inner wall surface, which are opposite to each other across the ion exchange resin molding 264, along the direction of the length thereof. The above arrangement enables an arbitrary curving in four directions, and the curving directions can be combined with each other to thereby enable a rotation.

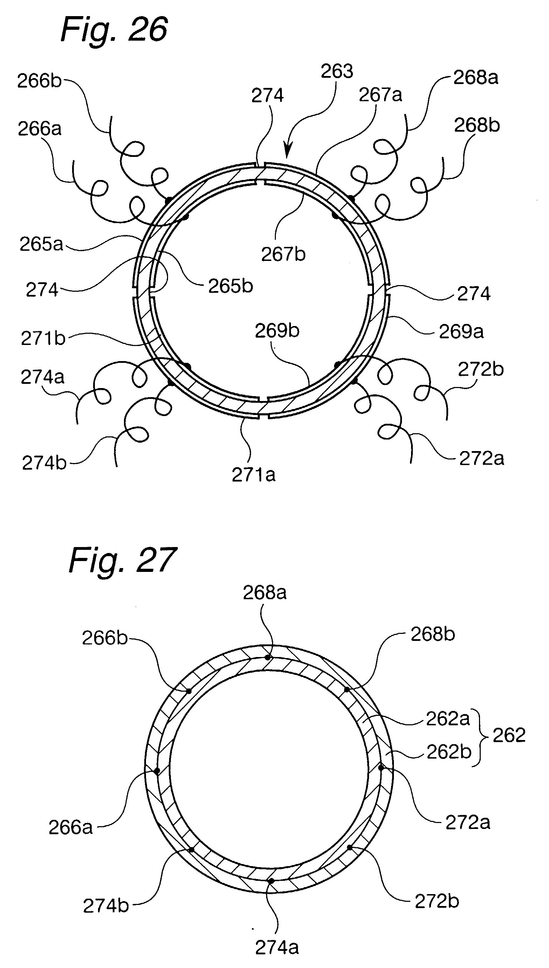

In this embodiment, a total of eight lead wires 266a, 266b, 268a, 268b, 272a, 272b, 274a, 274b run along the tube body 262. Referring to Figs. 26 and 27, the lead wires 266a, 266b, 268a, 268b, 272a, 272b, 274a, 274b are arranged in the junction interface of the inside tube 212a and the outside tube 212b to prevent the lead wires 266a, 266b, 268a, 268b, 272a, 272b, 274a, 274b from becoming a hindrance.

The lead wires 266a, 266b, 268a, 268b, 272a, 272b, 274a, 274b interposed between the inside tube 262a and the outside tube 262b can be held unmovable by the use of, for example, the double tube in which the outside tube 262b is composed of a thermally shrinkable resin.

Although in the foregoing embodiments the micromachine comprising the endoscope fitted with the microdevice has been described, it is naturally feasible to independently employ the microdevice in each medical field. Also, the endoscope body can be turned toward any arbitrary direction by arranging in the endoscope body the polymeric actuator as disposed in the microdevice of the present invention.

Moreover, although the micromachine comprising the microdevice of the present invention employed in the microsurgery of medical fields has been described in the foregoing embodiments, the microdevice of the present invention can be used in various sensors, repair tools and micromachines for carrying out, for example, the inspection or repair of plant piping systems, aircraft engine interiors, etc.

The process for producing an actuator element according to the present invention enables obtaining an actuator element which has a simple structure, can be easily miniaturized, exhibits a high response speed, exhibits a large displacement extent and can operate with low electric power.

Therefore, when the actuator element produced by the process of the present invention is joined to the front end of the guide member body of the microdevice, the actuator element can be arbitrarily and positively curved (deformed) by manipulation by means of the operation control unit. Accordingly, the microdevice capability of guiding a medical instrument for microsurgery, such as scissors, forceps, a snare, a laser knife or a spatula, any of various sensors or a mechanical tool connected to the front end of the guide member can be enhanced. As a result, it is feasible to effect the guidance thereof toward target site in any arbitrary direction, which can be speedily and easily performed without the need to have expertise.

Moreover, the actuator element produced by the process of the present invention can suitably be used in other items, for example, high-frequency vibration micropumps; health devices such as an auxiliary-power massage machine for rehabilitation; industrial appliances such as a hygrometer, a hygrometer control device, a software manipulator, a submersible valve and a software conveyor; submersible mobiles such as a goldfish and seaweeds; and hobby goods such as a moving fishing bait and a propeller fin.

The process for producing a lead wire according to the present invention, because it is only required to coat the surface of the lead wire substrate with a conductive solution, enables continuously providing conductive lines efficiently by simple procedure without the need to use a large apparatus. Furthermore, a highly conductive lead wire can be provided thereby, in which the bonding strength between conductive lines and substrate surface is excellent, the conductive lines being excellent in flexibility, being excellent in the follow-up to substrate elongation and not peeled from the substrate surface by the insertion or push in intricate or branched minute target sites or the pull therefrom or by the rotational torque.

In the employment of construction in which the lead wire substrate is in tubular form, the lead wire per se can be used as a guide, such as a medical tube or a medical wire, for a catheter, an endoscope, etc., so that a medicinal liquid path can be ensured and a size miniaturization can be attained.

In the fitting of the above lead wire in a fine microdevice, the lead wire fitting operation is easy to thereby ensure high productivity.

When the process for producing a lead wire according to the present invention is used and, also, the working jig therefor is used, in the irradiation working, the lead wire substrate is fitted and immobilized in the fixing channel provided in the irradiation working jig, and the surface, exposed from the irradiation working jig, of the lead wire substrate is subjected to the irradiation working. Thus, insulating zones can be formed by irradiation with, for example, laser with the lead wire substrate accurately positioned and fixed, so that mutual short circuiting of the conductive lines can be avoided.

Further, in the use of the microdevice and micromachine of the present invention, the polymeric actuator joined to the front end of the guide member body can be arbitrarily and positively curved (deformed) by the manipulation by means of the operation control unit. Accordingly, the microdevice capability of guiding a medical instrument for microsurgery, such as scissors, forceps, a snare, a laser knife or a spatula, any of various sensors or a mechanical tool connected to the frond end of the guide member can be enhanced, so that it is feasible to effect the guidance thereof toward target site in any arbitrary direction, which can be speedily and easily performed without the need to have expertise.

Still further, not only can a size miniaturization and a diameter reduction can be attained but also an excellent response can be realized by the construction of the polymeric actuator from the ion exchange resin molding and the electrode.

Therefore, in the technology of microsurgery such as ophthalmosurgery, peritoneoscopic surgery or microangiosuturing surgery, the application of the microdevice and micromachine fitted therewith according to the present invention to a medical instrument such as forceps, scissors, a clamp, a snare, a laser knife, a spatula or a clip enables minimizing the pain inflicted on patients at the time of examination or treatment and relieving the physical and psychic burden on patients. Furthermore, the application of the microdevice and micromachine fitted therewith according to the present invention to, for example, any of various sensors or a repair tool for use in the inspection or repair of a piping system or engine interior conducted in plants, such as power generating facilities, and mechanical systems, such as aircraft engines enables effectively performing such work without wasting labor and time.

## Claims

1. A process for producing an actuator element, said actuator element comprising an ion exchange resin molding and metal electrodes, formed in mutually insulated relationship on surface of the ion exchange resin molding, said ion exchange resin molding adapted to be curved or deformed upon application of a potential difference between the metal electrodes while the ion exchange resin molding is in hydrous state, which process comprises the steps of:
(1) causing the ion exchange resin molding to adsorb a metal complex in an aqueous solution, and
(2) reducing the adsorbed metal complex with a reducing agent so that a metal is precipitated on surface of the ion exchange resin molding to thereby form a metal electrode.

2. The process as claimed in claim 1, wherein a gold or platinum complex is used as the metal complex.

3. The process as claimed in claim 1 or 2, wherein, upon formation of the metal electrode, the ion exchange resin molding has its counter ion replaced by at least one cation selected from the group consisting of Li⁺, Na⁺ and Cu²⁺.

4. A process for producing a lead wire, comprising the steps of:
coating surface of a lead wire substrate of an insulating material with a conductive solution to thereby form a conductive thin-film layer on the surface, and
irradiating the lead wire substrate, in one direction therealong, with light so that the conductive thin-film layer is removed by the irradiation working to thereby form insulating zones and thus form a plurality of mutually electrically insulated conductive lines along the lead wire substrate.

5. The process as claimed in claim 4, wherein the coating with the conductive solution is performed by a dip coating.

6. The process as claimed in claim 4 or 5, wherein the conductive solution is a conductive solution of Au or Ag.

7. The process as claimed in any of claims 4 to 6, wherein the lead wire substrate is in tubular form.

8. The process as claimed in any of claims 4 to 7, wherein the irradiation working is performed by laser.

9. The process as claimed in any of claims 4 to 8, wherein, in the irradiation working, the lead wire substrate is fitted and fixed in a fixing channel provided in an irradiation working jig, and surface of the lead wire substrate, exposed from the irradiation working jig, is subjected to the irradiation working.

10. The process as claimed in claim 9, wherein use is made of the irradiation working jig comprising a pair of base frames arranged upside and downside, said base frames having matching faces provided with fixing channels, said base frames provided with irradiation slits extending from upper and lower surfaces of the base frames to the fixing channels, and wherein the lead wire substrate is fixed between the fixing channels by closing the base frames together and is at its surface subjected to irradiation working through the irradiation slits.

11. The process as claimed in claim 9, wherein the irradiation working jig comprising four base frames arranged upside right, upside left, downside right and downside left, said base frames having matching faces provided with fixing channels, said base frames provided with irradiation slits extending from upper, right, lower and left surfaces of the base frames to the fixing channels, and wherein the lead wire substrate is fixed between the fixing channels by closing the base frames together and is at its surface subjected to irradiation working through the irradiation slits.

12. The process as claimed in any of claims 4 to 8, wherein, in the irradiation working, both ends of the lead wire substrate are fixed by fixing clamps provided on an irradiation working jig; a surface of the lead wire substrate along its length is subjected to irradiation working; the fixing clamps are rotated to thereby rotate the lead wire substrate by a predetermined angle; and a surface of the lead wire substrate along its length is subjected to irradiation working.

13. The process as claimed in any of claims 4 to 12, wherein the irradiation working is conducted under such a numerical control that the irradiation working is carried out by scanning the surface of the lead wire substrate along its length on the basis of data inputted in advance in conformity with the configuration of the lead wire substrate.

14. A jig for irradiation working for use in producing a lead wire, comprising a plate base frame having its upper surface provided with a fixing channel, said fixing channel adapted to fix a lead wire substrate so that a surface of the lead wire substrate is subjected to irradiation from upside.

15. The working jig as claimed in claim 14, wherein the fixing channel is defined by a fixed plate fastened to an upper surface of the base frame and a channel width regulating plate arranged, movably toward the fixed plate, on the upper surface of the base frame.

16. A working jig comprising a pair of base frames arranged upside and downside,
said base frames having matching faces provided with fixing channels, said fixing channels adapted to fix a lead wire substrate therebetween by closing the base frames together,
said base frames provided with irradiation slits extending from upper and lower surfaces of the base frames to the fixing channels, said irradiation slits adapted to perform irradiation working of a surface of the lead wire substrate therethrough.

17. A working jig comprising four base frames arranged upside right, upside left, downside right and downside left,
said base frames having matching faces provided with fixing channels, said fixing channels adapted to fix a lead wire substrate therebetween by closing the base frames together,
said base frames provided with irradiation slits extending from upper, right, lower and left surfaces of the base frames to the fixing channels, said irradiation slits adapted to perform irradiation working of a surface of the lead wire substrate therethrough.

18. A working jig comprising a pair of fixing clamps capable of synchronous rotation for fixing both ends of a lead wire substrate, which is adapted to, after irradiation working of a surface of the lead wire substrate along its length, rotate the fixing clamps to thereby rotate the lead wire substrate by a predetermined angle for enabling further irradiation working of a surface of the lead wire substrate along its length.

19. A microdevice comprising:
a slender guide member body;
a therapeutic tool connected to a front end of the guide member body;
a polymeric actuator arranged in the vicinity of part of the connection; and
an actuator operation control unit electrically connected to the actuator through a lead wire extending along a longitudinal direction of the guide member body,
wherein the polymeric actuator comprises an ion exchange resin molding and, provided in positions interposing the ion exchange resin molding, at least one pair of electrodes, and
wherein an arrangement is made such that the ion exchange resin molding is deformed by selective application of a voltage through the lead wire to the pair of electrodes by means of the operation control unit so that the therapeutic tool connected to the front end of the guide member body can be oscillated in any arbitrary direction.

20. A micromachine comprising a tubular slender guide member body and, fitted in an internal space thereof, the microdevice of claim 19, wherein an arrangement is made such that the microdevice can be protruded through an opening made at a front end of the slender guide member body.
